# EUROPÄISCHE PATENTSCHRIFT

(11) **EP 1 284 120 B1**
(45) Veröffentlichungstag und Bekanntmachung des Hinweises auf die Patenterteilung: **11.05.2016**
(21) Anmeldenummer: 02008567.6
(22) Anmeldetag: 16.04.2002
(51) Int. Cl.: A61B 1/00

(54) **Einweg-Endoskopmantel**
Disposable endoscope sheath
Gaine à usage unique pour un endoscope

(30) Priorität: 09.08.2001 DE 10139153
(43) Veröffentlichungstag der Anmeldung: 19.02.2003
(73) Patentinhaber: INGOSCOPE SYSTEMS GmbH, 10999 Berlin (DE)
(72) Erfinder: Herrmann, Ingo F., Prof. Dr., 81479 München (DE)
(74) Vertreter: Manitz, Finsterwald & Partner GbR

(56) Entgegenhaltungen:
- DE-A- 19 906 191
- US-A- 4 721 097
- US-A- 5 429 596
- US-A- 5 489 256
- US-A- 5 846 183

## Beschreibung

Die Erfindung betrifft das Gebiet der Endoskopie, insbesondere im Bereich der mikroinvasiven Chirurgie, wie beispielsweise der Biopsie, der Laserchirurgie oder der Kryochirurgie.

Aktuelle Studien belegen, dass bei Endoskopen, die zu gastroenterologischen Zwecken eingesetzt worden sind, trotz nachfolgender Reinigung und Desinfektion mit einer Häufigkeit von bis zu annähernd 50 Prozent Kontaminationen durch Fäkalkeime oder Keime der "Pseudomonaden / Nonfermenter"-Gruppe nachgewiesen werden können. Diese Restkontaminationen können eine nicht verantwortbare Infektionsgefahr für den nachfolgend mit dem betreffenden Endoskop behandelten Patienten bedeuten.

Es ist eine Aufgabe der Erfindung, die Gefahr einer Infektionsübertragung auf einen Patienten durch ein Endoskop, das zuvor bei einem infizierten Patienten eingesetzt worden ist, zu beseitigen oder zumindest zu verringern.

Diese Aufgabe wird durch einen Einwegmantel gelöst, der zur Kopplung eines Endoskops mit einem Arbeitsgerät für einen einmaligen Gebrauch vorgesehen ist, wobei der Einwegmantel wenigstens einen Endoskopkanal zur Aufnahme des Endoskops aufweist.

Dem liegt die Erkenntnis zugrunde, dass Hohlräume, die an bekannten Endoskopen beispielsweise zur Aufnahme von Arbeitsgeräten vorgesehen sind, besonders schwierig zu reinigen sind und demzufolge eine besonders hohe Gefahr einer Infektionsübertragung bedeuten.

Durch die Verwendung eines Einwegmantels wird ermöglicht, dass derartige Hohlräume oder Kanäle ausschließlich an einem Wegwerfteil vorgesehen sind, das das Endoskop und das Arbeitsgerät für einen einmaligen Gebrauch am Patienten aufzunehmen vermag und nach dem einmaligen Gebrauch - anstelle einer Reinigung - entsorgt wird. Durch Verwendung dieses Einwegmantels kann also auf den Einsatz von Endoskopen mit einem eigenen Arbeitskanal oder einem eigenen Spül- oder Saugkanal verzichtet werden, und stattdessen können sogenannte Kompaktendoskope eingesetzt werden, die keine Hohlräume oder Kanäle besitzen. Ein derartiges Kompaktendoskop kann auf übliche, beispielsweise chemo-thermische Weise sterilisiert werden, wobei die Gefahr von Restkontaminationen beseitigt oder zumindest erheblich reduziert ist.

Einwegmäntel für Endoskope, die über einen Endoskopkanal und über einen separaten Spülkanal verfügen, sind beispielsweise aus US-A-5,846,183 oder US-A-4,721,097 bekannt.

Der Einsatz des Einwegmantels erfolgt derart, dass zunächst das Endoskop und das Arbeitsgerät, gegebenenfalls mit weiteren Instrumenten, in den Einwegmantel eingeführt werden, wobei insbesondere das distale Ende des Endoskops über das distale Ende des Einwegmantels hinausstehen kann. Sodann wird der Einwegmantel gemeinsam mit dem Endoskop und dem Arbeitsgerät in die betreffende Körperöffnung des Patienten eingeführt. Nach der gewünschten Diagnose oder Behandlung wird der Einwegmantel mit dem Endoskop und dem Arbeitsgerät der Körperöffnung wieder entnommen. Das Endoskop und das Arbeitsgerät werden von dem Einwegmantel getrennt. Dieser wird entsorgt, wohingegen das Endoskop und das Arbeitsgerät gereinigt werden.

Diese Anwendungsweise hat den Vorteil, dass das Einführen des Einwegmantels in die Körperöffnung durch das Endoskop unterstützt werden kann, nämlich indem mittels des Endoskops beziehungsweise des darin üblicherweise enthaltenen Bowdenzugs eine gegebenenfalls erforderliche Krümmung des Einwegmantels herbeigeführt wird, und indem mittels des üblicherweise vorhandenen Bildübertragungskanals des Endoskops der innerhalb des Körpers zurückzulegende Weg beobachtet wird.

Dieser Vorteil wird selbstverständlich auch dann erzielt, wenn zunächst der Einwegmantel gemeinsam lediglich mit dem Endoskop in die Körperöffnung eingeführt wird, während das Arbeitsgerät erst nachfolgend bei bereits positioniertem Einwegmantel nachgeführt wird. Je nach Anwendungsfall ist es auch möglich, zunächst lediglich den Einwegmantel in die betreffende Körperöffnung einzusetzen und erst danach des Endoskop und das Arbeitsgerät nachzuführen.

Dies bietet den Vorteil, dass zum einen bereits vorhandene Endoskope weiterhin eingesetzt werden können und zum anderen dennoch die Gefahr von Restkontaminationen verringert ist. Außerdem gewährleistet das erfindungsgemäße Prinzip eines Einwegmantels mit einem oder mehreren innenliegenden Kanälen zur Aufnahme von Endoskop, Arbeitsgerät oder sonstigen Utensilien eine hohe Anpassungsfähigkeit hinsichtlich aller Arten von endoskopischen Anwendungen.

Ein weiterer Vorteil hinsichtlich der erwünschten Hygiene besteht darin, dass aufgrund der Ummantelung des Endoskops und des Arbeitsgeräts durch den Einwegmantel Ablagerungen von Schleim, Sekreten oder sonstigen Körperflüssigkeiten an dem Endoskop beziehungsweise Arbeitsgerät vermieden oder zumindest verringert werden, was die nachfolgende rückstandsfreie Reinigung dieser Instrumente noch weiter vereinfacht.

Ferner kann für die Untersuchung und mikroinvasive Behandlung des Gallengangs oder des Bauchspeiclaeldrüsengangs auf den Einsatz eines bekannten, jedoch mechanisch vergleichsweise komplizierten sogenannten Albarran-Hebels verzichtet werden, da der erfindungsgemäße Einwegmantel einen parallelen, jedoch unabhängig voneinander betätigbaren Einsatz eines Endoskops und eines Arbeitsgeräts ermöglicht.

Bei einer bevorzugten Ausführungsform dient der Endoskopkanal - als kombinierter Endoskop-/Arbeitskanal - zur zusätzlichen Aufnahme des Arbeitsgeräts. Mit anderen Worten sind das Endoskop und das Arbeitsgerät innerhalb eines gemeinsamen Kanals vorgesehen, und sie können innerhalb dieses gemeinsamen Kanals vorzugsweise unabhängig voneinander bewegt werden.

Für diese Ausführungsform ist es ferner bevorzugt, wenn der Endoskop-/Arbeitskanäl einen länglichen, insbesondere einen im wesentlichen ovalen Innenquerschnitt aufweist. Dadurch können das Endoskop und das Arbeitsgerät, die üblicherweise jeweils einen im wesentlichen kreisrunden Querschnitt besitzen, nebeneinander liegend aufgenommen werden, wobei diese beiden Instrumente selbst bei einer Relativbewegung zueinander ihre benachbarte Anordnung grundsätzlich beibehalten.

Es ist möglich, das Endoskop und das Arbeitsgerät, die in einen derartigen gemeinsamen Endoskop-/Arbeitskanal eingeführt werden sollen, nach dem sogenannten Huckepack-Prinzip miteinander zu verbinden. Hierfür kann an dem Endoskop, beispielsweise am Distalende eine Schlinge oder Schlaufe vorgesehen sein, durch die das Arbeitsgerät geführt und gegebenenfalls in seiner Relativlage fixiert wird. Eine Huckepack-Verbindung zwischen Endoskop und Arbeitsgerät kann beispielsweise auch dadurch verwirklicht werden, dass diese beiden Instrumente mittels entlang der jeweiligen Längsrichtung angeordneter Magnetstreifen und/oder mittels eines distalen Einhakelements bzw. Gegenstücks temporär koppelbar sind.

Alternativ oder zusätzlich zu dieser Huckepack-Anordnung von Endoskop und Arbeitsgerät ist es auch möglich, innerhalb des Endoskop-/ Arbeitskanals wenigstens einen Führungssteg vorzusehen, durch den die Anordnung von Endoskop und Arbeitsgerät relativ zueinander stabilisiert wird. Mit anderen Worten kann der Einwegmantel im Querschnitt eine oder mehrere Einkerbungen beziehungsweise Einschnürungen aufweisen. Der genannte Führungssteg beziehungsweise die Führungsstege können sich abschnittsweise oder entlang der gesamten Länge des Einwegmantels erstrecken.

Alternativ zu der genannten Ausbildung eines gemeinsamen Endoskop-/Arbeitskanals kann ein vom Endoskopkanal separater Arbeitskanal vorgesehen sein, in den das Arbeitsgerät einzuführen ist. Diese Ausführungsform bietet den Vorteil, dass das Endoskop und das Arbeitsgerät völlig unabhängig voneinander innerhalb des Einwegmantels bewegt werden können, ohne dass die Gefahr einer gegenseitigen Beeinflussung oder Behinderung oder die Gefahr einer unbeabsichtigten Veränderung der Relativlage besteht.

Bei dieser Ausführungsform ist es bevorzugt, wenn der Endoskopkanal und/oder der Arbeitskanal - entsprechend dem jeweils üblichen Außenquerschnitt von Endoskop und Arbeitsgerät - jeweils einen im wesentlichen kreisrunden Innenquerschnitt besitzt.

Ungeachtet der möglichen Trennung von Endoskopkanal und Arbeitskanal ist es bevorzugt, wenn das Endoskop und das Arbeitsgerät unabhängig voneinander relativ zueinander und/oder relativ zu dem Einwegmantel entlang der Längsrichtung des Einwegmantels bewegt werden können.

Es ist ferner bevorzugt, wenn wenigstens eine Fixiereinrichtung, beispielsweise eine Fixierschraube oder eine Schiebeklemme, vorgesehen ist, durch die die Lage des Arbeitsgeräts relativ zu dem Einwegmantel zeitweise arretiert werden kann. Auf diese Weise kann eine Stabilisierung herbeigeführt werden, beispielsweise wenn mit dem Endoskop - in fester Lage - ein mit dem Arbeitsgerät durchgeführter mikrochirurgischer Eingriff beobachtet werden soll.

Ferner können bei einer Weiterbildung der Erfindung zusätzliche Arbeitskanäle vorgesehen sein, beispielsweise zur Aufnahme einer oder mehrerer Lasereinheiten.

Der erfindungsgemäße Einwegmantels weist wenigstens einen Spülkanal auf, durch den eine Flüssigkeit (beispielsweise Wasser) oder ein Gas (beispielsweise Luft) zu einer Auslassöffnung am Distalende des Einwegmantels übertragen, beispielsweise gepumpt werden kann. Dieser Spülkanal kann beispielsweise dazu verwendet werden, eine am Distalende des Endoskops vorhandene Bildaufnahmeoptik beständig mit Wasser zu spülen, um Sichtbehinderungen zu verhindern. Der Spülkanal kann auch zur Einfuhr von Druckluft, beispielsweise in den Magen verwendet werden. Es ist ein besonderer Vorteil der Erfindung, dass ein derartiger, gegebenenfalls vorhandener Spülkanal Teil des Einwegmantels ist, so dass bakterielle Verunreinigungen, die sich innerhalb des Spülkanals anlagern, keinesfalls zu einer Infektion eines nachfolgend behandelten Patienten führen können.

Es ist bevorzugt, wenn das proximale Ende dieses Spülkanals eine Verbindungseinrichtung aufweist, durch die der Spülkanal an den betreffenden Fluidspender, beispielsweise eine Pumpe oder einen Spülbehälter, angeschlossen werden kann. Die Realisierung einer derartigen Verbindungseinrichtung ist besonders einfach, falls ein Schlauch vorgesehen ist, der zum einen die Verbindungseinrichtung aufweist und zum anderen innerhalb des Spülkanals geführt ist oder in diesen übergeht. Es ist auch möglich, an dem proximalen Ende des Spülkanals beziehungsweise des genannten Schlauchs einen Anschlussflansch, ein Gewinde oder einen Bajonettverschluss vorzusehen, die eine Verbindung mit einem Standardanschluss des betreffenden Fluidspenders ermöglichen.

Außerdem ist es bevorzugt, wenn der Spülkanal wenigstens eine Verschlusseinrichtung, insbesondere wenigstens ein Ventil zum temporären Verschließen des Spülkanals besitzt. Beispielsweise kommen Einwegventile in Frage, die in den Einwegmantel integriert sind und vorzugsweise am proximalen Ende des Einwegmantels beherbergt sind.

Alternativ hierzu kann der Spülkanal wenigstens eine Aufnahmeeinrichtung aufweisen, die zur Aufnahme einer Mehrweg-Verschlusseinrichtung dient. Mit anderen Worten kann vorgesehen sein, dass eine - vergleichsweise hochwertige und deshalb teure - Verschlusseinrichtung zum temporären Verschließen des Spülkanals mehrfach und somit für mehrere verschiedene Einwegmäntel verwendet wird. In diesem Fall ist es bevorzugt, dass - aus den erläuterten Gründen der Hygiene - eine räumliche Trennung des Spülkanalinneren und der Mehrweg-Verschlusseinrichtung realisiert ist. Diese Trennung kann beispielsweise durch eine zwischen Spülkanal und Mehrweg-Verschlusseinrichtung angeordnete flexible Membran verwirklicht werden.

Alternativ oder zusätzlich zu dem genannten Spülkanal kann der Einwegmantel wenigstens einen Saugkanal aufweisen, der am distalen Ende des Einwegmantels mündet und dort zum Absaugen von Flüssigkeiten oder Gasen dient.

Entsprechend der vorgenannten Weiterbildung des Spülkanals kann dieser Saugkanal eine Verbindungseinrichtung zum Anschluss an eine Absaugpumpe, eine Verschlusseinrichtung zum temporären Verschließen des Saugkanals und/oder eine Aufnahmeeinrichtung zur Aufnahme einer mehrfach verwendbaren Verschlusseinrichtung besitzen.

Falls sowohl ein Spülkanal als auch ein Saugkanal vorgesehen sind, die zum Freispülen der Optik eines Bildaufnahmeendes des Endoskops entlang einer Querrichtung dienen, so ist es bevorzugt, wenn die distale Öffnung des Spülkanals und die distale Öffnung des Saugkanals relativ zueinander in einer Gegenüberstellung angeordnet sind.

Im übrigen ist es auch möglich, einen kombinierten Spül-/Saugkanal vorzusehen, der eine interne Zweiteilung aufweist, um einen Fluidfluss in entgegengesetzten Richtungen zu ermöglichen.

Alternativ oder zusätzlich zu der erläuterten Aufnahme eines Arbeitsgeräts in einen Endoskop-/Arbeitskanal oder einen separaten Arbeitskanal des Einwegmantels kann bei einer vorteilhaften Weiterbildung der Erfindung eine insbesondere lösbare Ankopplung eines Arbeitsgeräts an die Außenseite des Einwegmantels vorgesehen sein. Zu diesem Zweck kann der Einwegmantel wenigstens eine Außenkopplungseinrichtung besitzen. Bei dieser Weiterbildung dient der Einwegmantel also als Adapter zur Kopplung des innerhalb des Einwegmantels aufgenommenen Endoskops mit dem an die Außenseite des Einwegmantels angekoppelten Arbeitsgeräts.

Die genannte Außenkopplungseinrichtung kann insbesondere eine Schnappverbindungseinrichtung aufweisen, die beispielsweise eine elastische Schnappklammer oder eine elastische Schnapprinne zum Umgreifen des Arbeitsgeräts besitzt.

Alternativ oder zusätzlich kann die Außenkopplungseinrichtung eine Magnetkopplungseinrichtung aufweisen, die sich entlang der Längsachse des Einwegmantels durchgehend oder lediglich entlang eines oder mehrerer Längsabschnitte erstreckt. Insbesondere kann die Magnetkopplungseinrichtung lediglich an dem distalen Ende des Einwegmantels vorgesehen sein, um dort eine Ankopplung des Arbeitsgeräts zu ermöglichen.

Vorzugsweise ist die Magnetkopplungseinrichtung durch einen Permanentmagneten gebildet. Die Magnetkopplungseinrichtung kann im Wesentlichen einstückig oder in Pulverform in den Einwegmantel integriert sein. Es ist von besonderem Vorteil, wenn die Magnetkopplungseinrichtung als austauschbare Mehrwegeinheit vorliegt, die für einen mehrmaligen Gebrauch einem Einwegmantel entnommen und bei einem anderen Einwegmantel ein- oder aufgesetzt werden kann. Eine derartige Ausgestaltung ermöglicht eine mehrfache Nutzung einer hochwertigen und bezüglich der weiteren Bestandteile des Einwegmantels vergleichbaren teuren Magnetkopplungseinrichtung.

Alternativ oder zusätzlich hierzu kann die Außenkopplungseinrichtung auch eine Klettverschlusseinrichtung, eine Klebeverbindungseinrichtung oder eine Schienen-/ Nutverbindungseinrichtung aufweisen.

Die genannte Außenkopplungseinrichtung ist vorzugsweise zu einer derart lösbaren Kopplung des Arbeitsgeräts an den Einwegmantel ausgebildet, dass der Einwegmantel - nachdem der Einwegmantel mit dem darin aufgenommenen Endoskop und dem daran angekoppelten Arbeitsgerät in die betreffende Körperöffnung eingeführt worden ist - gemeinsam mit dem Endoskop aus der Körperöffnung entnommen werden kann, während das dabei oder zuvor von dem Einwegmantel gelöste Arbeitsgerät noch in der Körperöffnung verbleibt. Zu diesem Zweck ist beispielsweise die Klammer- oder Schnappkraft der genannten Schnappklammer oder Schnapprinne hinreichend gering gewählt, um ein seitliches Freigeben des angekoppelten Arbeitsgeräts zu ermöglichen. In entsprechender Weise kann auch die Kopplungskraft einer Magnetkopplungseinrichtung so gering gewählt sein, dass das Arbeitsgerät abschnittsweise von dem Einwegmantel abgezogen werden kann.

Vorzugsweise ist die genannte Außenkopplungseinrichtung lediglich entlang eines oder mehrerer Umfangsabschnitte des Einwegmantels vorgesehen. Dadurch kann die Winkellage vorbestimmt werden, in der das Arbeitsgerät an den Einwegmantel bezüglich dessen Längsachse angekoppelt wird. Dies ist insbesondere bei Ankopplung mehrerer Arbeitsgeräte von Vorteil, die eine bestimmte Winkellage relativ zueinander oder zu einer vorbestimmten Krümmungsrichtung des Einwegmantels oder des darin aufgenommenen Endoskops einnehmen sollen.

Weiterhin ist es bevorzugt, wenn seitens des anzukoppelnden Arbeitsgeräts wenigstens eine Gegenkopplungseinrichtung vorgesehen ist, die mit der genannten Außenkopplungseinrichtung zusammenwirkt. Hierfür kann es beispielsweise im Falle der genannten Schnappverbindungseinrichtung genügen, an dem Arbeitsgerät auf einfache Weise einen im Wesentlichen runden Querschnitt vorzusehen, der mittels der Schnappverbindungseinrichtung des Einwegmantels leicht umgriffen werden kann. Falls die Außenkopplungseinrichtung des Einwegmantels durch einen Permanentmagneten gebildet ist, genügt es, als Gegenkopplungseinrichtung für das Arbeitsgerät eine Ausbildung aus einem magnetischen Material vorzusehen.

Vorzugsweise ist die Außenkopplungseinrichtung dergestalt ausgebildet, dass sie ein Verschieben oder sonstigen Bewegen des angekoppelten Arbeitsgeräts bezüglich der Längsachse des Einwegmantels ermöglicht. Dadurch kann mit dem Arbeitsgerät in flexibler Weise agiert werden, beispielsweise um an unterschiedlichen Stellen innerhalb des Körpers pH-Messwerte aufzunehmen oder Gewebeproben zu entnehmen.

Es ist bevorzugt, wenn an dem Einwegmantel eine Fixiereinrichtung vorgesehen ist, durch die die Lage des Arbeitsgeräts entlang der Längsachse des Einwegmantels fixiert werden kann, um eine feste Relativanordnung des Arbeitsgeräts bezüglich des Einwegmantels und insbesondere des darin aufgenommenen Endoskops zu gewährleisten. Diese Fixiereinrichtung kann durch die Außenkopplungseinrichtung selbst gebildet sein.

Die Fixiereinrichtung kann beispielsweise eine arretierbare Fixierklammer am proximalen Ende des Einwegmantels aufweisen, so dass der behandelnde Arzt leicht eine temporäre Fixierung herbeiführen oder diese wieder auflösen kann. Die Fixiereinrichtung kann auch einen Permanentmagneten oder einen wahlweise aktivierbaren Elektromagneten aufweisen, der entlang des Einwegmantels oder an dessen distalem Ende angeordnet ist. Ferner kann als Fixiereinrichtung am distalen Ende des Einwegmantels eine Einhakeinrichtung vorgesehen sein, durch die das distale Ende des Arbeitsgerät zeitweise am Einwegmantel befestigt werden kann. Beispielsweise kann eine derartige Einhakeinrichtung als Hinterschneidungsansatz ausgebildet sein, der ein entsprechendes Mitnehmerelement am Arbeitgerät bezüglich der Einführrichtung des Einwegmantels umgreift.

Gemäß einer vorteilhaften Ausführungsform besitzt der Einwegmantel wenigstens zwei Umfangserhöhungen, die - einander gegenüberstehend - eine Außenkopplungseinrichtung seitlich umgeben und dadurch einen im Querschnitt konkaven Umfangsabschnitt einschließen, innerhalb dessen ein Arbeitsgerät zur Ankopplung an die Außenkopplungseinrichtung aufgenommen werden kann. Derartige Umfangserhöhungen an der Außenseite des Einwegmantels dienen also zur zusätzlichen seitlichen Stabilisierung eines angekoppelten Arbeitsgeräts.

Ferner ist es bevorzugt, wenn der Einwegmantel mehrere, insbesondere zwei oder drei Außenkopplungseinrichtungen aufweist, die eine Ankopplung einer entsprechenden Anzahl von Arbeitsgeräten an die Außenseite des Einwegmantels ermöglichen.

Insbesondere bei Vorhandensein mehrerer Außenkopplungseinrichtungen ist es bevorzugt, wenn wenigstens eine Außenkopplungseinrichtung für eine Ankopplung eines Arbeitsgeräts dergestalt ausgebildet ist, dass das distale Ende des Arbeitsgeräts an einem Längsabschnitt des Einwegmantels noch vor dem distalen Ende des Einwegmantels angeordnet und dort vorzugsweise fixierbar ist. Bei dieser Ausführungsform können mehrere Arbeitsgeräte an den Einwegmantel und somit an ein darin aufgenommenes Endoskop angekoppelt werden, wobei die distalen Enden der Arbeitsgeräte bezüglich der Längsachse des Einwegmantels bzw. des Endoskops auf unterschiedlicher Höhe angeordnet sind. Eine derartige Anordnung ermöglicht beispielsweise die Anwendung der somit gebildeten Diagnoseeinheit zur Somnoskopie, also zur Beobachtung des Patienten während des Schlafs. Zum Beispiel kann mittels mehrerer pH-Messsensoren der pH-Wert an unterschiedlichen Stellen der Speiseröhre eines schlafenden Patienten gemessen werden, ohne dass es aufgrund einer zwischenzeitlichen Verschiebung der Einheit aus Endoskop, Einwegmantel und angekoppelten pH-Messsensoren zu einer Verfälschung der Messergebnisse kommt.

Bei einer weiteren vorteilhaften Ausführungsform der erläuterten Ausgestaltung des Einwegmantels mit einer Außenkopplungseinrichtung ist - zumindest wahlweise - ein Ankopplungsschlauch vorgesehen, der als Adapter zur Ankopplung eines beliebigen Arbeitsgeräts an den Einwegmantel dient und zu diesem Zweck wenigstens eine mit der Außenkopplungseinrichtung zusammenwirkende Gegenkopplungseinrichtung aufweist. Vorzugsweise ist auch ein derartiger Ankopplungsschlauch als ein Einwegteil vorgesehen, um die Einhaltung der erläuterten hygienemedizinischen Erfordernisse zu gewährleisten.

Bei der erläuterten Ausgestaltung des Einwegmantels mit einer Außenkopplungseinrichtung kann ein integriertes Spülsystem und/ oder ein Saugsystem vorgesehen sein, wie im Zusammenhang mit dem Einwegmantel mit Arbeitskanal oder Endoskop-/Arbeitskanal bereits erläutert. Alternativ oder zusätzlich kann zumindest ein an die Außenkopplungseinrichtung anzukoppelndes Arbeitsgerät als ein Saug-/Spülschlauch mit wenigstens einem Saug-/Spülkanal ausgebildet sein, also mit einem Saugkanal, einem Spülkanal, einem kombinierten Saug-/Spülkanal oder sowohl mit einem Spülkanal als auch mit einem hiervon separaten Saugkanal. Mit anderen Worten ist der Saug-/Spülkanal nicht unbedingt in den Einwegmantel integriert, sondern er kann an die Außenseite des Einwegmantels angekoppelt werden.

Vorzugsweise ist bei dieser Ausführungsform eine distale Fixiereinrichtung an der Außenkopplungseinrichtung oder an dem Einwegmantel vorgesehen, durch die der Saug-/ Spülschlauch bezüglich der Längsachse des Einwegmantels dergestalt fixiert wird; dass das distale Ende des Saug-Spülkanals einen vorbestimmten Abstand relativ zu dem distalen Ende des Einwegmantels oder des darin aufgenommenen Endoskops einnimmt. Eine derartige Fixiereinrichtung kann also gewährleisten, dass der Saug-/Spülkanal stets mit der Endoskopspitze abschließt, so dass immer eine hinreichende Reinigung der Endoskopoptik möglich ist. Diese distale Fixiereinrichtung kann beispielsweise eine Fixierklammer, eine Fixierverengung oder einen mit einem magnetischen Gegenelement zusammenwirkenden Permanentmagneten oder wahlweise aktivierbaren Elektromagneten aufweisen, und sie kann insbesondere durch die Außenkopplungseinrichtung selbst gebildet sein. Ferner kann als derartige distale Fixiereinrichtung die bereits erläuterte Einhakeinrichtung vorgesehen sein.

Das Vermeiden des erläuterten unerwünschten Verrutschens eines angekoppelten Saug-/ Spülschlauchs bezüglich des distalen Endes eines im Einwegmantel befindlichen Endoskops kann auch dadurch erreicht oder unterstützt werden, dass der als anzukoppelndes Arbeitsgerät vorgesehene Saug-/Spülschlauch in Längsrichtung elastisch dehnbar ist. Dadurch kann auch bei einer Krümmung des Endoskops bzw. des Einwegmantels das distale Ende des Saug-/Spülkanals weiterhin bündig mit der Endoskopspitze bzw. mit deren Optik abschließen.

Sofern als anzukoppelndes Arbeitsgerät ein externer Saug-/ Spülschlauch vorgesehen ist, ist es ferner bevorzugt, wenn dieser Saug-/Spülschlauch durch eine proximale und eine distale Außenkopplungseinrichtung dergestalt bezüglich der Längsachse des Einwegmantels fixiert werden kann, dass bei einer Krümmung des Einwegmantels der Saug-/Spülschlauch gespannt oder gedehnt wird. Mit anderen Worten wird der Saug-/Spülschlauch mittels der Außenkopplungseinrichtungen bezüglich der Längsrichtung des Einwegmantels fixiert. Dies hat zur Folge, dass bei einer Krümmung des Einwegmantels, beispielsweise mittels eines Bowdenzugs des in den Einwegmantel aufgenommenen Endoskops, eine Dehnung oder ein Spannen des Saug-/Spülschlauchs entlang dessen Längsachse bewirkt wird. Ein derartiges Dehnen oder Spannen trägt dazu bei, ein Kollabieren des Saug-/Spülkanals zu verhindern. Eine Zugspannung eines derartigen Saug-/Spülschlauchs verursacht nämlich eine Stabilisierung der Seitenwände dergestalt, dass ein Abknicken des Kanals und somit ein Blockieren der Saug-/Spülwirkung verhindert wird. Dies ist insbesondere für das Absaugen von besonderer Bedeutung, da nicht unbedingt stets eine Flüssigkeit innerhalb des Saugkanals geführt ist, die den Saugkanal gegen ein Kollabieren stützt.

Alternativ oder zusätzlich zu den Ausführungsformen des erfindungsgemäßen Einwegmantels mit innenliegende Endoskop-/Arbeitskanal, mit separatem Arbeitskanal oder mit Außenkopplungseinrichtung kann bei einer vorteilhaften Weiterbildung ein magnetisches Zusammenwirken eines Arbeitsgeräts durch den Einwegmantel hindurch mit dem darin aufgenommenen Endoskop vorgesehen sein. Zu diesem Zweck ist wenigstens ein Umfangsabschnitt des Einwegmantels, der den Endoskopkanal begrenzt, als ein Kopplungsübertragungsabschnitt ausgebildet, der ein magnetisches Zusammenwirken einer Magnethalteeinrichtung des in dem Einwegmantel befindlichen Endoskops einerseits mit einer Magnetgegeneinrichtung des Arbeitsgeräts andererseits erlaubt. Bei dieser Weiterbildung ist also eine magnetische Außenkopplung des Arbeitsgeräts vorgesehen, wobei das magnetische Zusammenwirken nicht direkt mit dem Einwegmantel, sondern mit dem darin aufgenommenen Endoskop erfolgt. Ein Vorteil dieser Weiterbildung besteht darin, dass der Einwegmantel keine eigene Magnetkopplungseinrichtung aufweisen muss.

Der genannte Kopplungsübertragungsabschnitt des Einwegmantels ist vorzugsweise durch eine Verdünnung der Außenhaut des Einwegmantels oder durch eine Umfangsöffnung des Einwegmantels gebildet. Diese Ausgestaltungen bewirken, dass die Magnethalteeinrichtung des Endoskops und die Magnetgegeneinrichtung des Arbeitsgeräts räumlich besonders nahe aneinander angeordnet sein können und deshalb besonders wirkungsvoll zusammenwirken können.

Bei der genannten Magnethalteeinrichtung und Magnetgegeneinrichtung kann es sich um einen Permanentmagneten und ein magnetisierbares Material handeln, oder umgekehrt.

Der Kopplungsübertragungsabschnitt kann sich im Wesentlichen entlang der gesamten Länge des Einwegmantels erstrecken. Alternativ hierzu können ein oder mehrere Köpplungsübertragungsabschnitte an dem Einwegmantel ausgebildet sein, die sich jeweils lediglich entlang eines begrenzten Längsabschnitts des Einwegmantels erstrecken. Insbesondere kann ein Kopplungsübertragungsabschnitt zumindest an dem distalen Ende des Einwegmantels vorgesehen sein, um dort ein magnetisches Ankoppeln des Arbeitsgeräts zu ermöglichen.

Als anzukoppelndes Arbeitsgerät kann insbesondere ein Saug-/Spülschlauch mit wenigstens einem Saug-/Spülkanal vorgesehen sein, wie vorstehend im Zusammenhang mit der Außenkopplungseinrichtung erläutert. Alternativ oder zusätzlich kann bei dieser Weiterbildung mit Kopplungsübertragungsabschnitt der Einwegmantel einen oder mehrere integrierte Saug-/Spülkanäle besitzen, wie vorstehend bereits erläutert.

Weiterhin ist es von Vorteil, wenn der Einwegmantel wenigstens zwei Umfangserhöhungen besitzt, die den Kopplungsübertragungsabschnitt seitlich umgeben und einen im Querschnitt konkaven Umfangsabschnitt einschließen, der zur stabilisierenden Aufnahme des Arbeitsgeräts dienen kann.

Ferner kann auch bei dieser Weiterbildung ein Ankopplungsschlauch vorgesehen sein, der zur Aufnahme des Arbeitsgeräts ausgebildet ist und die genannte Magnetgegeneinrichtung aufweist. In diesem Fall muss also das Arbeitsgerät nicht unbedingt magnetisch ausgebildet sein. Dieser Ankopplungsschlauch kann als Einwegteil ausgebildet sein.

Bei einer besonders vorteilhaften Ausgestaltung der erläuterten Weiterbildung mit Außenkopplungseinrichtung oder der Weiterbildung mit Kopplungsübertragungsabschnitt besitzt der Einwegmantel zwei - vorzugsweise integrierte - Saug-/Spülkanäle, die bezüglich des Endoskopkanals dergestalt angeordnet sind, dass der Einwegmantel an seiner Außenseite zwischen den Saug-/ Spülkanälen einen im Querschnitt konkaven Umfangsabschnitt zur Aufnahme des Arbeitsgeräts aufweist. Mit anderen Worten ist die Anordnung der Saug-/ Spülkanäle derart gewählt, dass das an der Außenseite des Einwegmantels anzukoppelnde Arbeitsgerät dazwischen eingebettet werden kann. Die Längsachsen der beiden Saug-/ Spülkanäle können bezüglich der Längsachse des Endoskopkanals dabei beispielsweise einen Winkel zwischen 30° und 120°, insbesondere einen Winkel von etwa 45°, 60° oder 90° relativ zueinander einnehmen.

Auch bei der erläuterten Weiterbildung des Einwegmantels mit Kopplungsübertragungsabschnitt kann an dem Einwegmantel eine distale Fixiereinrichtung der erläuterten Art vorgesehen sein, um das distale Ende eines Arbeitsgeräts oder eines Saug-/Spülschlauchs temporär oder dauerhaft bezüglich des distalen Endes des Einwegmantels oder des darin befindlichen Endoskops zu fixieren.

Die eingangs genannte Aufgabe wird auch durch einen Einwegmantel gelöst, der für einen einmaligen Gebrauch ausgebildet ist und zumindest einen Endoskopkanal zur Aufnahme eines Endoskops sowie wenigstens einen Saug-/Spülkanal besitzt. Bei diesem Einwegmantel ist also nicht unbedingt die Aufnahme oder Ankopplung eines Arbeitsgeräts vorgesehen. Stattdessen ist in den Einwegmantel wenigstens ein Saugkanal, ein Spülkanal, oder ein kombinierter Saug-/Spülkanal integriert. Dadurch kann ein herkömmliches Endoskop, das über keinen eigenen Saug-/Spülkanal verfügt, nachträglich um einen derartigen Saug-/Spülkanal ergänzt werden.

Auch bei einem derartigen Einwegmantel sind die bereits erläuterten hygienemedizinischen Vorteile gewährleistet, da der hinsichtlich der Gefahr von Restkontaminationen besonders kritische Saug-/Spülkanal seitens des Einwegmantels vorgesehen ist, der als Wegwerfteil konzipiert ist. Durch Verwendung dieses Einwegmantels kann also wiederum ein sogenanntes Kompaktendoskop ohne eigene Hohlräume oder Kanäle eingesetzt werden, das beispielsweise auf chemo-thermische Weise mit hinreichender Zuverlässigkeit sterilisiert werden kann. Für Anwendungen, bei denen ein oder mehrere Saug-/Spülkanäle benötigt werden, wird das Endoskop zuerst in den Endoskopkanal des Einwegmantels eingeführt. Sodann wird der Einwegmantel gemeinsam mit dem Endoskop in die betreffende Körperöffnung, beispielsweise den Rachen des Patienten eingeführt. Nach der Diagnose oder Behandlung wird das Endoskop von dem Einwegmantel getrennt. Dieser kann dann entsorgt werden.

Die nachstehend erläuterten Ausführungsformen beziehen sich auf alle der vorstehend genannten Ausgestaltungen und Weiterbildungen des erfindungsgemäßen Einwegmantels.

Hinsichtlich der Form des erfindungsgemäßen Einwegmantels ist es bevorzugt, wenn dieser einen im wesentlichen kreisrunden oder ovalen Außenquerschnitt aufweist. In diesem Fall findet der Einwegmantel nämlich besonders flexiblen Einsatz hinsichtlich der jeweiligen Körperöffnung in die das Endoskop mit samt Arbeitsgerät eingeführt werden soll. Vor allem jedoch werden bei dieser Ausgestaltung Verletzungen des Patienten, wie sie bei unbeabsichtigten Verdrehungen des Endoskops beziehungsweise des Einwegmantels auftreten können, besonders wirkungsvoll vermieden.

Alternativ hierzu kann der Einwegmantel einen Außenquerschnitt besitzen, der dem Querschnitt des menschlichen Nasengangs entspricht und beispielsweise im Wesentlichen die Form eines Dreiecks mit abgerundeten Ecken besitzt. In diesem Fall kann der Einwegmantel mit darin aufgenommenem Endoskop - im Gegensatz zur herkömmlichen Gastroskopie - einem Patienten über einen Nasengang in den Rachen und die Speiseröhre eingeführt werden.

Da der Einwegmantel vorzugsweise einen möglichst gleichförmigen Außenquerschnitt besitzt und andererseits alle Kanäle, wie Endoskopkanal, Arbeitskanal und Spül-/Saugkanal, vollständig innerhalb des Einwegmantels verlaufen, kann es erforderlich sein, den Innenraum des Einwegmantels mit einem Füllmaterial zu versehen.

Vorzugsweise ist der Einwegmantel aus einem flexiblen Material, insbesondere Kunststoff gebildet. Bevorzugt handelt es sich um elastisches, also rückfederndes Material, da hierdurch Krümmungen, die beispielsweise mittels des Bowdenzugs des Endoskops herbeigeführt werden, selbständig in eine geradlinige Ausrichtung rückgeführt werden können.

Eine elastische Ausgestaltung des Einwegmantels kann auch dergestalt vorgesehen sein, dass dieser hinsichtlich eines Umfangsabschnitts oder hinsichtlich seines gesamten Umfangs elastisch, also rückfedernd dehnbar ist. Dadurch kann der Einwegmantel für Endoskope und/oder Arbeitsgeräte unterschiedlichen Durchmessers auf flexible Weise eingesetzt werden, wobei die Rückstellkräfte stets ein enges Anliegen der Kanalwände an dem aufgenommenen Endoskop bzw. Arbeitsgerät gewährleisten. Eine derartige elastische Ausbildung des Umfangs des Einwegmantels kann entlang eines begrenzten Längsabschnitts des Einwegmantels oder entlang im Wesentlichen der gesamten Länge des Einwegmantels vorgesehen sein. In letzterem Fall kann der Einwegmantel also nach Art einer Strumpfhülle über das Endoskop gezogen werden. Bei einer derartigen Ausgestaltung besitzen der Arbeitskanal und/ oder ein Saug-/Spülkanal vorzugsweise eine höhere Festigkeit, insbesondere eine dickere Wandstärke, um die erforderliche Stabilität für das Einführen eines Arbeitsgeräts bzw. für die Zirkulation eines Fluids zu gewährleisten.

Gemäß einer vorteilhaften Ausführungsform des erfindungsgemäßen Einwegmantels besitzt der zur Aufnahme des Arbeitsgeräts vorgesehene Kanal, also der Endoskop-/Arbeitskanal oder der separate Arbeitskanal, eine Versteifung, die sich vorzugsweise entlang eines Umfangsabschnitts erstreckt, der bei einer vorgesehenen Krümmung oder Abwinklung des Einwegmantels mittels des darin geführten Endoskops krümmungsaußenseitig verläuft. Aufgrund einer derartigen Versteifung sind vergleichsweise starke Beugungen des Einwegmantels beispielsweise bis zu 90° möglich, wobei das Arbeitsgerät dennoch innerhalb des zugeordneten Kanals ungehindert axial bewegt werden kann. Falls eine derartige Versteifung vorgesehen ist, kann für den Einwegmantel ein Material einer vergleichsweise geringen Elastizität verwendet werden, so dass der Arbeitskanal kollabiert, wenn sich kein Arbeitsgerät in dem Kanal befindet. Eine derartige Ausgestaltung hat den Vorteil eines geringeren Platzbedarfs des Einwegmantels.

Weiterhin ist es hinsichtlich der Ausgestaltung des Einwegmantels bevorzugt, wenn der Endoskopkanal, der Arbeitskanal und/oder der kombinierte Endoskop-/Arbeitskanal entlang eines oder mehrerer Längsabschnitte des Einwegmantels wenigstens eine seitliche Öffnung besitzt. Eine derartige seitliche Öffnung ermöglicht eine Beobachtung, eine Messung oder eine Gewebeentnahme an dem betreffenden Längsabschnitt des Einwegmantels, ohne dass das distale Ende des Einwegmantels mitsamt Endoskop und Arbeitsgerät an den betreffenden Abschnitt der Körperöffnung geführt werden muss.

Hinsichtlich der Ausgestaltung des Einwegmantels ist es außerdem bevorzugt, wenn dieser aus einem transparenten Material gebildet ist. Auch in diesem Fall ist eine Beobachtung oder zumindest eine Beleuchtung einer umgebenden Körperregion durch den Einwegmantel hindurch möglich, ohne dass das Endoskop oder das betreffende Arbeitsgerät unbedingt zu einer Kanalöffnung am distalen Ende oder an einen besonderen Längsabschnitt des Einwegmantels bewegt werden muss.

Eine besonders kostengünstige Herstellung des Einwegmantels ist möglich, falls dieser einstückig aufgebaut ist. In diesem Fall besteht auch nicht die Gefahr eines unbeabsichtigten Loslösens von einzelnen Bestandteilen innerhalb des Körpers des Patienten. Zumindest sollte der äußere Umfangsbereich des Einwegmantels einstückig und möglichst glatt ausgebildet sein, um bei Bewegungen des Einwegmantels innerhalb der Körperöffnung nicht durch Kantenübergänge die Gefahr von Verletzungen herbeiführen zu können.

Hinsichtlich einer kostengünstigen, insbesondere endlosen Herstellung des Einwegmantels sowie eines möglichst flexiblen Einsatzes des Einwegmantels ist es bevorzugt, wenn die in dem Einwegmantel enthaltenen Kanäle am Distalende bündig zueinander, also auf gleicher Höhe abschließen.

Das distale Ende des Einwegmantels besitzt eine Fixiereinrichtung, durch die die Lage des distalen Endes des Endoskops relativ zu dem Einwegmantel fixiert werden kann. Dadurch können beispielsweise unerwünschte Relativbewegungen während einer Diagnose oder einer Behandlung vermieden werden. Die genannte Fixiereinrichtung weist einen Verengungsabschnitt auf, entlang dessen der Innendurchmesser des Endoskopkanals verengt ist, um mit dem in dem Endoskopkanal eingeführten Endoskop einen Kraftschluss zu bilden und dadurch unerwünschte Verschiebungen des Endoskops zu verhindern.

Zusätzlich kann die Fixiereinrichtung einen Permanentmagneten aufweisen, der am distalen Ende des Einwegmantels dem Endoskopkanal benachbart angeordnet ist. Ein derartiger Permanentmagnet kann - beispielsweise durch Erhöhung des Reibungskraftschlusses - eine Fixierung des distalen Endes des Endoskops bewirken, falls dieses magnetisch ist. In entsprechender Weise kann anstelle des Permanentmagneten am distalen Ende des Einwegmantels ein magnetischer Abschnitt vorgesehen sein. In diesem Fall kann eine Fixierung des im Endoskopkanal befindlichen Endoskops beispielsweise dadurch bewirkt werden, dass ein Elektromagnet am distalen Ende des Endoskops aktiviert wird.

Als Fixiereinrichtung am distalen Ende des Endoskopkanals ist ein Einschnürbund vorgesehen, der für einen Kraftschluss mit dem Endoskop und somit für eine erhöhte Reibung sorgt, so dass ein Verschieben des in dem Einschnürbund gefangenen Endoskops erschwert ist.

Hinsichtlich des proximalen Endes des Einwegmantels ist es bevorzugt, wenn dieses eine Abschlussverbreiterung aufweist, durch die ein unbeabsichtigtes vollständiges Eindringen des Einwegmantels in die Körperöffnung verhindert wird.

Außerdem ist anzumerken, dass die Erfindung sich auch auf ein Endoskopiesystem bezieht, bei dem der erfindungsgemäße Einwegmantel gemeinsam mit einem - hinsichtlich des jeweiligen Durchmessers - geeigneten Endoskop und insbesondere auch einem geeigneten Arbeitsgerät zur Verfügung steht.

Weitere Ausführungsformen der Erfindung sind in den Unteransprüchen genannt.

Die Erfindung wird nachfolgend beispielhaft unter Bezugnahme auf die Zeichnungen erläutert; in diesen zeigen:
- Fig. 1: eine schematische Perspektivansicht des Distalendes eines Einwegmantels mit eingesetztem Endoskop und Arbeitsgerät,
- Fig. 2 bis 5: Frontansichten des Distalendes jeweils einer weiteren Ausführungsform eines Einwegmäntels,
- Fig. 6a, 6b und 6c: eine Frontansicht, eine Seitenansicht beziehungsweise eine Draufsicht auf das Distalende einer weiteren Ausführungsform eines Einwegmantels,
- Fig. 7: eine Querschnittsdraufsicht auf das Proximalende eines Einwegmantels,
- Fig. 8: eine Querschnittsseitenansicht des Proximalendes eines Einwegmantels,
- Fig. 9a, 9b und 9c: vergrößerte Detailansichten verschiedener Ausführungsformen des Bereichs IX gemäß Fig. 8,
- Fig. 10: eine vergrößerte Detailansicht einer alternativen Ausführungsform des Bereichs X gemäß Fig. 8,
- Fig. 11 und 12: jeweils eine schematische Perspektivansicht eines erfindungsgemäßen Einwegmantels,
- Fig. 13a und 13b: Querschnittsansichten des Einwegmantels gemäß Fig. 11 bzw. Fig. 12,
- Fig. 14: eine Frontansicht auf das Distalende eines Kompaktendoskops,
- Fig. 15a bis 15g: Querschnittsansichten weiterer Ausführungsformen des Einwegmantels gemäß Fig. 11,
- Fig. 16: eine Querschnittsansicht eines weiteren Einwegmantels,
- Fig. 17: eine schematische Perspektivansicht eines weiteren Einwegmantels,
- Fig. 18a und 18b: Querschnittsansichten des Einwegmantels gemäß Fig. 17,
- Fig. 19: eine schematische Perspektivansicht eines weiteren Einwegmantels,
- Fig. 20: eine Querschnittsansicht des Einwegmantels gemäß Fig. 19,
- Fig. 21: eine schematische Perspektivansicht eines weiteren Einwegmantels,
- Fig. 22a und 22b: Frontansichten des Distalendes bzw. des Proximalendes des Einwegmantels gemäß Fig. 21,
- Fig. 23a bis 23e: Querschnittsansichten weiterer Ausführungsformen des Einwegmantels gemäß Fig. 21,
- Fig. 24a bis 24e: Querschnittsansichten verschiedener Ausführungsformen eines weiteren Einwegmantels,
- Fig. 25: eine schematische Perspektivansicht eines weiteren Einwegmantels, und
- Fig. 26a und 26b: Frontansichten des Distalendes bzw. des Proximalendes des Einwegmantels gemäß Fig. 25.

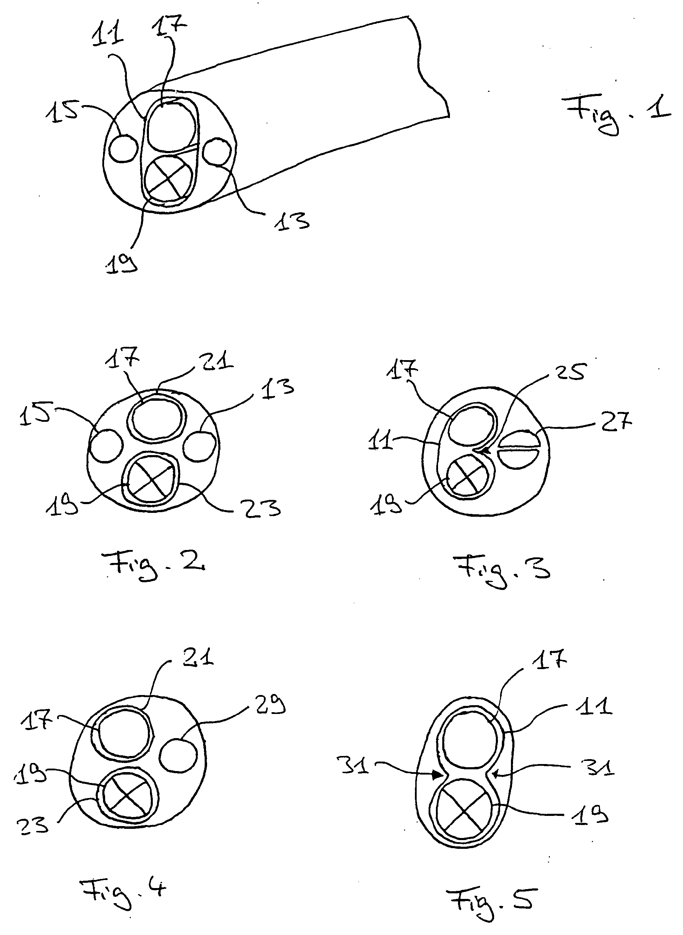

Fig. 1 zeigt das Distalende eines Einwegmantels. Zu erkennen ist die ovale Austrittsöffnung eines Endoskop-/Arbeitskanals 11, die runde Austrittsöffnung eines Spülkanals 13 und die runde Eintrittsöffnüng eines Saugkanals 15. Innerhalb des Endoskop-/Arbeitskanals 11 befinden sich benachbart zueinander ein Endoskop 17 mit rundem Außenquerschnitt und ein Arbeitsgerät 19, nämlich eine Biopsie-Zange. Der Spülkanal 13 und der Saugkanal 15 sind bezüglich des Endoskop-/Arbeitskanals 11 in einer Gegenüberstellung angeordnet.

Der Einwegmantel gemäß Fig. 1 ist als flexibler Kunststoffschlauch mit einer zylindrischen Grundform ausgebildet, wobei der Endoskop-/Arbeitskanal 11, der Spülkanal 13 und der Saugkanal 15 sich entlang des Einwegmantels parallel zueinander erstrecken. Die gezeigte distale Stirnseite des Einwegmantels erstreckt sich senkrecht zur Längsrichtung des Einwegmantels, so dass die Öffnungen der Kanäle 11, 13, 15 im wesentlichen bündig zueinander abschließen.

Zum Einsatz in der Gastroenterologie kann der in Fig. 1 gezeigte Einwegmantel eine Länge zwischen 50 und 90 cm und einen (größeren) Innendurchmesser des Endoskop-/Arbeitskanals 11 zwischen 8 und 12 mm aufweisen. Zur Verwendung speziell in der Kolonoskopie können die Länge zwischen 50 und 130 cm und der Innendurchmesser des Endoskop-/Arbeitskanals 11 beispielsweise 15 mm betragen.

Der Einwegmantel gemäß Fig. 1 dient zum einen zur parallelen Führung des Endoskops 17 und des Arbeitsgeräts 19. Zu diesem Zweck sind diese beiden Instrumente 17, 19 innerhalb des Einwegmantels grundsätzlich frei beweglich. Somit kann, wenn der Einwegmantel in eine Körperöffnung eingeführt worden ist und dort eine feste Lage beibehält, das Arbeitsgerät 19 aus dem distalen Ende des Einwegmantels zum Zwecke einer Probenentnahme herausgeschoben werden, und des Endoskop 17 kann zur gleichzeitigen Beobachtung dieser Probenentnahme relativ zu dem Einwegmantel und dem Arbeitsgerät 19 bewegt werden. Zu diesem Zweck ist das Endoskop 17 als sogenanntes Fibroskop ausgebildet, das heißt es besitzt einen Licht-/Bildübertragungskanal mit einer Bildaufnahmeoptik am distalen Ende. Ferner ist das Endoskop 17 mit einem, zwei oder vier Bowdenzügen ausgestattet, um Bewegungen in einer oder zwei Querrichtungen durchführen zu können und diese Querbewegungen auch auf den flexiblen Einwegmantel übertragen zu können.

Zum anderen kann über den Spülkanal 13 und den Saugkanal 15 eine Spülflüssigkeit aus dem distalen Ende des Einwegmantels ausgegeben beziehungsweise in dieses wieder eingesaugt werden, um die Bildaufnahmeoptik des Endoskops 17 von unerwünschten Ablagerungen freizuhalten.

Der Einwegmantel gemäß Fig. 1 besitzt den Vorteil, dass er das gemeinsame und parallele Führen von Endoskop 17 und Arbeitsgerät 19 gestattet, ohne dass eines dieser Instrumente 17, 19 einen eigenen Kanal besitzen muss. Der Einwegmantel kann nach der beschriebenen Verwendung entsorgt werden, während die - hohlraumfreien - Instrumente 17, 19 nach Gebrauch auf übliche Weise gereinigt werden. Die vor allem bei Instrumenten mit Hohlräumen bestehende Gefahr von Restkontaminationen ist dadurch erheblich verringert.

Die Ausführungsform gemäß Fig. 1 eignet sich insbesondere für eine gegenseitige Kopplung von Endoskop 17 und Arbeitsgerät 19 nach dem bereits genannten Huckepack-Prinzip.

Fig. 2 zeigt eine weitere Ausführungsform eines Einwegmantels. Hier ist das Endoskop 17 innerhalb eines eigenen Endoskopkanals 21 angeordnet, und für das Arbeitsgerät 19 ist ein separater Arbeitskanal 23 vorgesehen.

Bei der Ausführungsform gemäß Fig. 3 besitzt der kombinierte Endoskop-/Arbeitskanal 11 in Draufsicht eine ovale Grundform mit einem mittigen seitlichen Führungssteg 25, der sich im wesentlichen entlang der gesamten Länge des Einwegmantels erstreckt und die Relativlage von Endoskop 17 und Arbeitsgerät 19 stabilisiert. Außerdem ist bei der Ausführungsform gemäß Fig. 3 ein einziger, jedoch zweigeteilter Spül-/Saugkanal 27. vorgesehen.

Die Ausführungsform gemäß Fig. 4 besitzt einen eigenen Endoskopkanal 21, einen separaten Arbeitskanal 23 und einen Spülkanal, der als Druckluftkanal 29 dient.

Der Einwegmantel gemäß Fig. 5 besitzt einen ovalen Außenquerschnitt. Als einziger Kanal ist ein kombinierter Endoskop-/Arbeitskanal 11 vorgesehen, der einen im wesentlichen ovalen Innenquerschnitt mit zwei gegenüberliegenden Führungsstegen 31 aufweist.

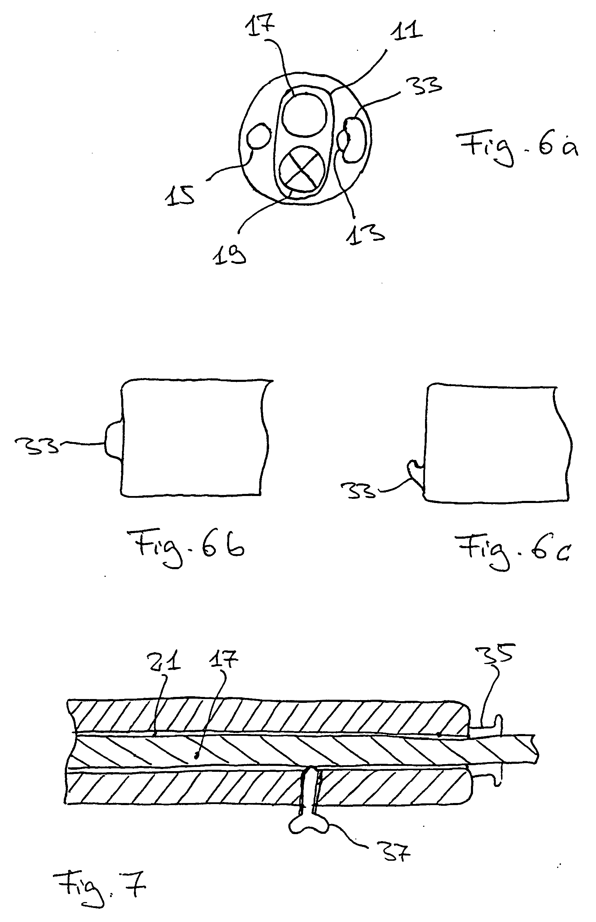

Der Einwegmantel gemäß Fig. 6a entspricht der Ausführungsform gemäß Fig. 1, wobei jedoch die distale Öffnung des Spülkanals 13 teilweise von einer Umlenkeinrichtung 33 überdeckt ist. Die Umlenkeinrichtung 33 ist durch einen Flügel gebildet, der vorzugsweise an der distalen Stirnseite des Einwegmantels angeformt ist (vgl. Fig. 6b und 6c). Die Umlenkeinrichtung 33 dient dazu, die aus dem Spülkanal 13 austretende Spülflüssigkeit über die Bildaufnahmeoptik des Endoskops 17 in Richtung des Saugkanals 15 zu lenken.

Fig. 7 zeigt eine Querschnitts-Draufsicht auf das proximale Ende eines erfindungsgemäßen Einwegmantels. Auch das proximale Ende ist im wesentlichen zylindrisch ausgebildet, wobei jedoch Spül-/Saugkanalfortsätze 35 ausgebildet sind, wie nachfolgend noch erläutert wird.

Innerhalb des Endoskopkanals 21 ist ein Teil eines Endoskops 17 zu erkennen. Das Endoskop 17 kann relativ zu dem Einwegmantel mittels einer seitlichen Fixierschraube 37 gegenüber einer unerwünschten Längsverschiebung gesichert werden.

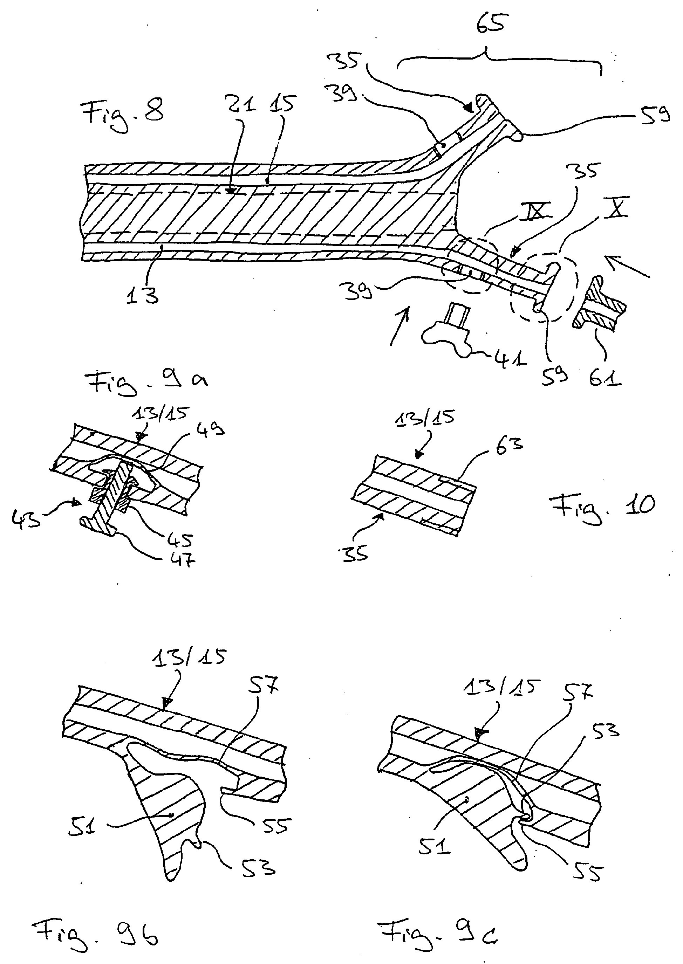

Fig. 8 zeigt eine Querschnitts-Seitenansicht des proximalen Endes des Einwegmantels gemäß Fig. 7. Hier sind die Spül-/Saugkanalfortsätze 35 zu erkennen, in denen der Spülkanal 13 beziehungsweise der Saugkanal 15 münden.

An den beiden Spül-/Saugkanalfortsätzen 35 ist jeweils eine Gewindeöffnung 39 vorgesehen, über die der betreffende Kanal 13 beziehungsweise 15 sich mittels einer Verschlussschraube 41 temporär verschließen lässt. Die Verschlussschraube 41 kann - ebenso wie der Einwegmantel - als Kunststoff-Wegwerfteil vorgesehen sein.

Alternativ zu dieser Art des Verschließens von Spülkanal 13 und Saugkanal 15 kann ein Mehrweg-Rastverschluss 43 vorgesehen sein, wie in der vergrößerten Detailansicht gemäß Fig. 9a gezeigt. Der Mehrweg-Rastverschluss 43 besitzt ein Grundelement 45, das in eine Einsatzöffnung des betreffenden Kanals 13 beziehungsweise 15 stabil eingesetzt werden kann. Über einen - nicht dargestellten - Ein- und Ausrastmechanismus ist das Grundelement 45 mit einem innenliegenden Verschlusselement 47 verbunden, das durch sukzessive Betätigung in den Kanal 13 beziehungsweise 15 eingebracht beziehungsweise herausgenommen werden kann, um den Kanal temporär zu verschließen oder wieder zu öffnen.

Ein direkter Kontakt des Mehrweg-Rastverschlusses 43 mit dem im Kanal 13 beziehungsweise 15 geführten Fluid wird vermieden, indem der Kanal 13 beziehungsweise 15 zwischen der genannten Einsatzöffnung und dem Kanalinneren eine Membran 49 besitzt, die einstückig mit der Kanalbegrenzung verbunden ist.

Fig. 9b zeigt in einer vergrößerten Detailansicht eine weitere Möglichkeit, den Spülkanal 13 beziehungsweise den Saugkanal 15 temporär zu verschließen. Zu diesem Zweck ist an dem Kanal 13 beziehungsweise 15 ein seitlicher Verschlusspfropfen 51 angeformt. Dieser kann in Richtung des Kanalinneren geschwenkt werden, so dass der Kanal 13 beziehungsweise 15 geschlossen ist, wie in Fig. 9c gezeigt. Dabei verrastet ein Rastelement 53 des Verschlusspfropfens 51 mit einem an dem Kanal 13 beziehungsweise 15 angeformten Gegenelement 55.

Auch bei dieser Ausführungsform ist zu jedem Zeitpunkt ein Austritt des in dem Kanal 13 beziehungsweise 15 geführten Fluids durch eine flexible Membran 57 ausgeschlossen.

Die bereits erläuterte Fig. 8 zeigt ferner, dass das jeweilige Ende der Spül-/Saugkanalfortsätze 35 als Anschlussflansch 59 ausgebildet ist, um das Verbindungsende 61 eines (nicht dargestellten) Spülbehälters oder einer Pumpe für den Zeitraum des Gebrauchs des Einwegmantels befestigen zu können.

Eine alternative Ausgestaltung des jeweiligen Endes der Spül-/Saugkanalfortsätze 35 ist in einer vergrößerten Detailansicht gemäß Fig. 10 gezeigt. Hier ist anstelle des Anschlussflansches 59 ein Außengewinde 63 vorgesehen, über das die Verbindung mit einem Spülbehälter oder einer Pumpe bewerkstelligt werden kann.

Zur Fig. 8 ist schließlich noch anzumerken, dass die seitliche Erstreckung der Spül-/Saugkanalfortsätze 35 als Abschlussverbreiterung 65 wirkt, die inhärent verhindert, dass der Einwegmantel unerwünscht vollständig in die Körperöffnung des Patienten eindringt.

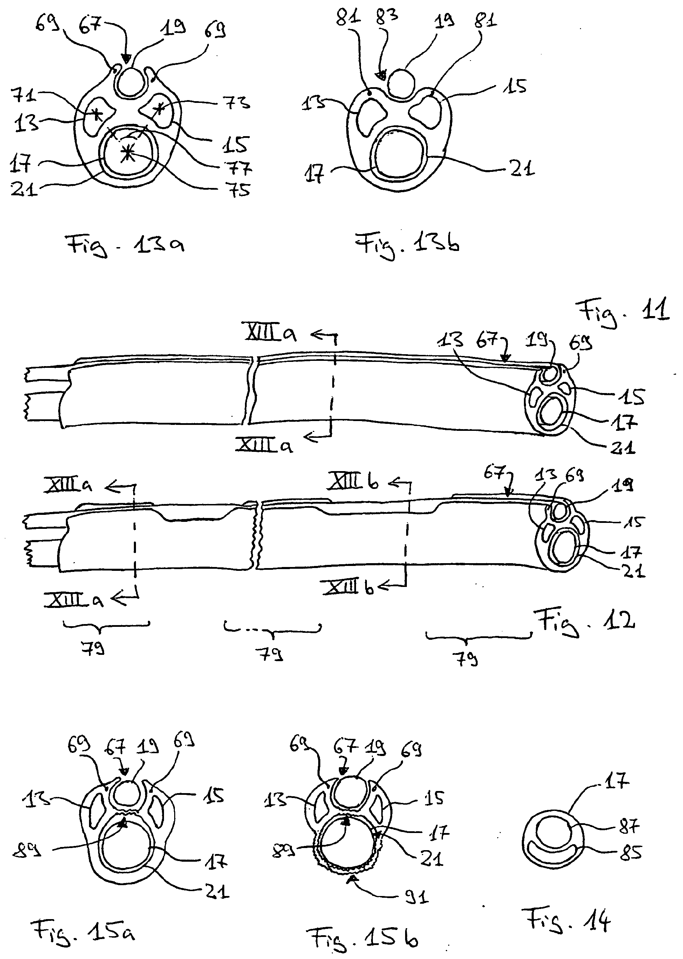

Fig. 11 zeigt in einer schematischen Perspektivansicht eines Einwegmantels, und insbesondere dessen distales Ende. Der Einwegmantel gemäß Fig. 11 besitzt einen Endoskopkanal 21, in dem ein Endoskop 17 längsbeweglich angeordnet ist. Ferner sind in dem Einwegmantel ein Spülkanal 13 und ein Saugkanal 15 integriert, deren Austrittsöffnungen am distalen Ende des Einwegmantels münden. Ein Umfangsabschnitt des Einwegmantels ist zu einer elastischen Schnapprinne 67 ausgebildet, die sich entlang der gesamten Länge des Einwegmantels erstreckt. Die Schnapprinne 67 dient als Schnappverbindungseinrichtung zur Ankopplung eines Arbeitsgeräts 19, beispielsweise einer pH-Metriesonde, einer Manometriesonde, einer Nährsonde oder einer Biopsie-Zange, an dem Einwegmantel und somit an das Endoskop 17. Das Arbeitsgerät 19 wird von zwei Seitenwänden 69 der Schnapprinne 67 umgriffen und ist somit innerhalb der Schnapprinne 67 längsbeweglich gefangen.

Der Einwegmantel gemäß Fig. 11 ist als flexibler Kunststoffschlauch ausgebildet. Er ermöglicht die Ankopplung des Arbeitsgeräts 19 an das Endoskop 17 sowie das Zuführen und Absaugen von Spülflüssigkeiten an seinem distalen Ende, ohne dass das Endoskop 17 oder das Arbeitsgerät 19 zu diesen Zwecken mit Hohlräumen versehen sein müssen. Aufgrund der erläuterten Kopplung kann das Arbeitsgerät 19 mittels des Endoskops 17 in eine Körperöffnung, beispielsweise über die Nase in den Rachenraum und nachfolgend in die Speiseröhre eines Patienten eingeführt werden, wobei gleichzeitig eine Beobachtung mittels des Endoskops 17 möglich ist und eine derartige Beobachtung durch permanentes Spülen der Optik des Endoskops 17 über den Spülkanal 13 und den Saugkanal 15 unterstützt werden kann. Für diese Verwendung kann das Arbeitsgerät 19 auf besonders einfache Weise mit dem Einwegmantel verbunden werden, da es von außen an den Einwegmantel bzw. in die Schnapprinne 17 angeklippt wird. Nach der beschriebenen Verwendung können das Arbeitsgerät 19 und das Endoskop 17 von dem Einwegmantel entfernt werden. Dieser wird entsorgt, und das Arbeitsgerät 19 und das Endoskop 17 werden auf chemo-thermische Weise gereinigt. Eine derartige Reinigung kann besonders gründlich erfolgen, da an dem Arbeitsgerät 19 und dem Endoskop 17 keine Hohlräume erforderlich sind, die sich entlang der jeweiligen Längsachse erstrecken.

Fig. 13a zeigt eine Querschnittsansicht des Einwegmantels gemäß Fig. 11 entlang der Ebene XIIIa-XIIIa. Diese Ansicht zeigt, dass das in die Schnapprinne 67 eingeschnappte Arbeitsgerät 19 von den beiden Seitenwänden 69 formschlüssig umgriffen wird. Der jeweilige Querschnitt des Spülkanals 13 und des Saugkanals 15 ist nicht kreisrund, sondern an den jeweiligen Außenumfang des Endoskopkanals 21, der Schnapprinne 67 und des Einwegmantels angepasst. Somit sind der Spülkanal 13 und der Saugkanal 15 letztlich dergestalt angeordnet, dass der Einwegmantel zwischen diesen Kanälen 13, 15 einen konkaven Umfangsabschnitt zur Aufnahme des Arbeitsgeräts 19 aufweist. Die Längsachse 71 des Spülkanals 13 und die Längsachse 73 des Saugkanals 15 nehmen bezüglich der Längsachse 75 des Endoskopkanals 21 einen Winkel 77 von ca. 60° relativ zueinander ein.

Der Durchmesser des Endoskopkanals 21 kann beispielsweise zwischen 3,7 und 4 mm betragen. Der Durchmesser des Spülkanals 13 oder des Saugkanals 15 kann beispielsweise zwischen 0,8 und 1 mm betragen. Der Außendurchmesser des in Fig. 11 gezeigten Einwegmantels kann beispielsweise zwischen 8 und 12 mm betragen.

Fig. 12 zeigt in einer schematischen Perspektivansicht eine ähnliche Ausführungsform eines Einwegmantels wie Fig. 11. Hier ist die als Außenkopplungseinrichtung dienende Schnapprinne 67 jedoch nicht entlang der gesamten Länge des Einwegmantels ausgebildet, sondern lediglich an mehreren Längsabschnitten 79 des Einwegmantels. Diese Unterteilung des Einwegmantels in mehrere Längsabschnitte mit bzw. ohne Schnapprinne 67 ermöglicht es, das distale Ende des Arbeitsgeräts 19 an einem Längsabschnitt ohne Schnapprinne 67 anzuordnen, so dass hier für das Arbeitsgerät 19 eine größere Bewegungsfreiheit beispielsweise für erwünschte Seitwärtsbewegungen oder ein größeres Gesichtsfeld für optische Beobachtungen besteht.

Fig. 13a zeigt wiederum eine Querschnittsansicht dieses Einwegmantels entlang der Ebene XIIIa-XIIIa. Fig. 13b zeigt eine Querschnittsansicht entlang der Ebene XIIIb-XIIIb, die einen Längsabschnitt des Einwegmantels gemäß Fig. 12 durchquert, an dem keine Schnapprinne 67 ausgebildet ist. Statt dessen sind die Wandungen des Spülkanals 13 und des Saugkanals 15 jeweils zu einer Umfangserhöhung 81 des Einwegmantels ausgebildet, so dass zwischen diesen beiden Umfangserhöhungen 81 ein konkaver Umfangsabschnitt 83 ausgebildet ist, in den das Arbeitsgerät 19 eingebettet ist.

Zu den Ausführungsformen gemäß Fig. 11 bis 13 ist noch anzumerken, dass der Spülkanal 13 und der Saugkanal 15 mit Kanalfortsätzen ausgestattet sein können, wie im Zusammenhang mit Fig. 7 bis 10 beschrieben. Insbesondere kann am jeweiligen proximalen Ende der Kanäle 13, 15 eine Verbindungseinrichtung zum Anschluss an eine Pumpe oder einen Spülbehälter, eine Verschlusseinrichtung zum temporären Verschließen des Kanals 13, 15, und/oder eine Aufnahmeeinrichtung zur Aufnahme einer Mehrweg-Verschlusseinrichtung vorgesehen sein.

Fig. 14 zeigt schematisch eine typische Frontansicht des distalen Endes eines Endoskops 17, wie es im Zusammenhang mit den Ausführungsbeispielen gemäß den Figuren Verwendung finden kann. Dieses Endoskop ist als Kompaktendoskop mit besonders einfachem Aufbau ausgebildet, und es besitzt demzufolge im Wesentlichen lediglich einen Lichtleiter 85 und eine Aufnahmeoptik 87, sowie gegebenenfalls einen oder mehrere Bowdenzüge (nicht gezeigt).

Fig. 15a bis 15g zeigen Querschnittsansichten von alternativen Ausführungsformen von Einwegmänteln mit wenigstens einer Außenkopplungseinrichtung, ähnlich der Querschnittsansicht gemäß Fig. 13a.

Bei dem Einwegmantel gemäß Fig. 15a ist der Umfangsabschnitt 89 des Endoskopkanals 21 zwischen dem Spülkanal 13 und dem Saugkanal 15 und somit zwischen den beiden Seitenwänden 69 der Schnapprinne 67 elastisch ausgebildet. Dadurch können Endoskope 17 unterschiedlichen Durchmessers in den Endoskopkanal 21 aufgenommen werden. Ebenso eignet sich die Schnapprinne 67 in flexiblerer Weise zur Ankopplung von Arbeitsgeräten 19 unterschiedlichen Durchmessers.

Fig. 15b zeigt eine Ausführungsform, bei der - in ähnlicher Weise wie bei Fig. 15a - ein Umfangsabschnitt 89 des Endoskopkanals 21 zwischen den Seitenwänden 69 der Schnapprinne 67, und zusätzlich ein bezüglich des Spülkanals 13 und des Saugkanals 15 gegenüberliegender Umfangsabschnitt 91 des Endoskopkanals 21 elastisch ausgebildet ist. Dadurch ist die Verwendung dieses Einwegmantels noch vielseitiger, was die Aufnahme von Endoskopen 17 unterschiedlichen Durchmessers betrifft.

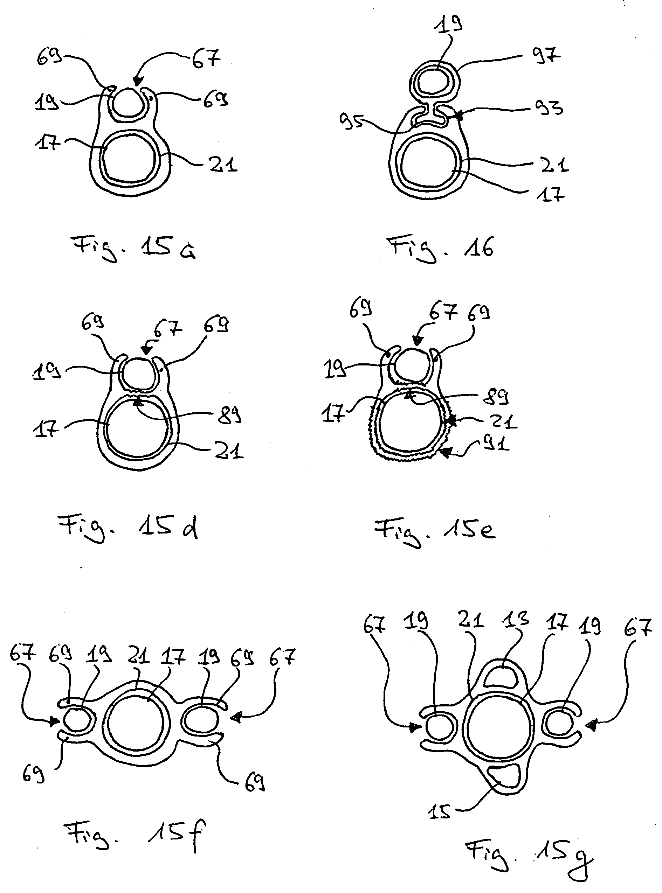

Fig. 15c zeigt eine Ausführungsform des Einwegmantels, bei der lediglich ein Endoskopkanal 21 und eine einzige Außenkopplungseinrichtung in Form einer Schnapprinne 67 zur Ankopplung eines Arbeitsgeräts 19 vorgesehen sind.

Fig. 15d zeigt eine Variante der Ausführungsform gemäß Fig. 15c, bei der der Umfangsabschnitt 89 zwischen den beiden Seitenwänden 69 der Schnapprinne 67 elastisch ausgebildet ist.

Fig. 15e zeigt eine Weiterbildung der Ausführungsform gemäß Fig. 15d, bei der - wie bei Fig. 15b - zusätzlich ein weiterer Umfangsabschnitt 91 elastisch ausgebildet ist, um Endoskope 17 unterschiedlichen Durchmessers aufnehmen zu können.

Fig. 15f zeigt eine Abwandlung der Ausführungsform gemäß Fig. 15c, bei der zwei Außenkopplungseinrichtungen in Form zweier einander gegenüberstehender Schnapprinnen 67 vorgesehen sind, in die jeweils ein Arbeitsgerät 19 eingeklippt werden kann.

Fig. 15g zeigt eine Weiterbildung der Ausführungsform gemäß Fig. 15f, bei der der Einwegmantel zusätzlich zu den beiden Außenkopplungseinrichtungen 67 einen Spülkanal 13 und einen Saugkanal 15 in einer gegenüberstehenden Anordnung aufweist.

Fig. 16 zeigt eine Querschnittsansicht einer weiteren Ausführungsform eines Einwegmantels, der - wie bei Fig. 15c - einen Endoskopkanal 21 und eine einzige Außenkopplungseinrichtung besitzt. Diese ist durch eine Nutverbindungseinrichtung 93 gebildet. In diese greift formschlüssig eine Schienenverbindungseinrichtung 95 eines Ankopplungsschlauchs 97 ein. Innerhalb des Ankopplungsschlauchs 97 befindet sich ein Arbeitsgerät 19.

Der Einwegmantel und der Ankopplungsschlauch 97 gemäß Fig. 16 dienen als Adapter zur Ankopplung des Arbeitsgeräts 19 an das in Fig. 16 gezeigte Endoskop 17. Indem das Arbeitsgerät 19 nicht direkt mit dem Einwegmantel verbunden wird, sondern die Ankopplung über den Ankopplungsschlauch 97 erfolgt, ist es möglich, zum einen unterschiedliche Ankopplungsschläuche 97 für Arbeitsgeräte 19 unterschiedlichen Durchmessers und zum anderen unterschiedliche Einwegmäntel für Endoskope 17 unterschiedlichen Durchmessers bereitzustellen, wobei - mittels der stets gleich ausgebildeten Nutverbindungseinrichtung 93 und Schienenverbindungseinrichtung 95 - stets eine Kopplungsverbindung zwischen dem Arbeitsgerät 19 und dem Endoskop 17 hergestellt werden kann. Es ist jedoch auch möglich, die gezeigte Schienenverbindungseinrichtung 95 direkt an dem Arbeitsgerät 19 vorzusehen.

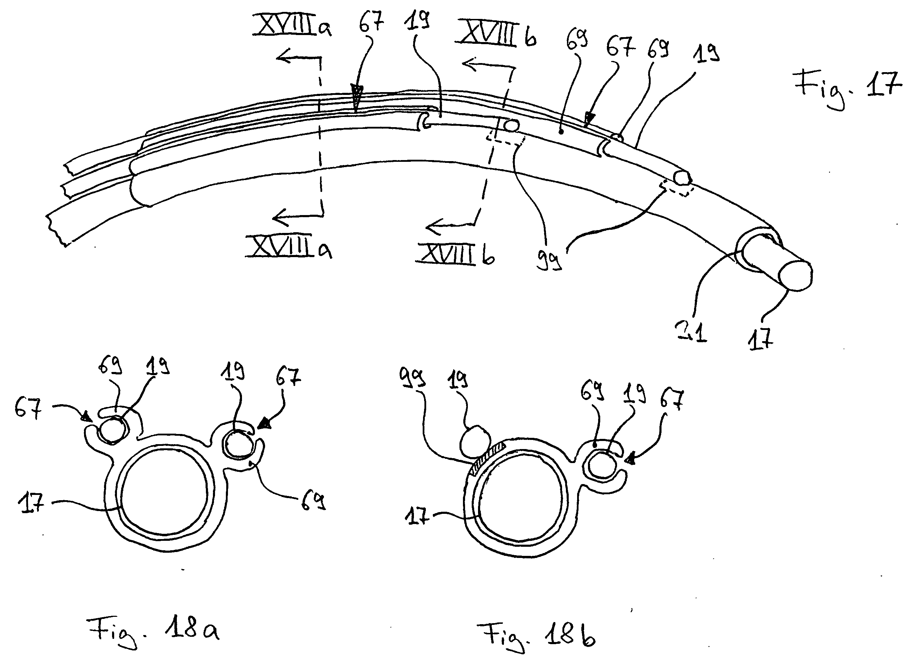

Fig. 17 zeigt eine schematische Perspektivansicht einer weiteren Ausführungsform eines Einwegmantels. Fig. 18a und 18b zeigen Querschnittsansichten hiervon entlang der Ebene XVIIIa-XVIIIa bzw. XVIIIb-XVIIIb gemäß Fig. 17.

Bei dieser Ausführungsform ist ein Endoskopkanal 21 zur Aufnahme eines Endoskops 17 vorgesehen. Ferner sind zwei Außenkopplungseinrichtungen jeweils in Form einer Schnapprinne 67 vorgesehen, die sich ausgehend von dem proximalen Ende des Einwegmantels entlang unterschiedlicher Längsabschnitte des Einwegmantels erstrecken. Innerhalb jeder Schnapprinne 67 ist ein Arbeitsgerät 19, beispielsweise ein pH-Messsensor geführt. Die Arbeitsgeräte 19 ragen aus dem distalen Ende der jeweiligen Schnapprinne 67 heraus, um beispielsweise zum Zwecke einer Somnoskopie Messungen oder Beobachtungen der Körperumgebung bei möglichst freier Messumgebung bzw. freiem Gesichtsfeld durchführen zu können.

Um die aus den Schnapprinnen 67 herausragenden distalen Enden der Arbeitsgeräte 19 bezüglich der Längsachse des gezeigten Einwegmantels zu fixieren, sind innerhalb eines jeweiligen. Längsabschnitts des Einwegmantels in Verlängerung der Schnapprinnen 67 zwei Magnetkopplungseinrichtungen 99 vorgesehen. Diese sind beispielsweise durch Permanentmagneten gebildet, die in den Einwegmantel integriert sind (vgl. Fig. 18b) und die magnetischen, beispielsweise metallisch ausgebildeten Arbeitsgeräte 19 anziehen. Die gezeigten Magnetkopplungseinrichtungen 99 bilden somit Fixiereinrichtungen, durch die die Lage der Arbeitsgeräte 19 relativ zu der Längsachse des gezeigten Einwegmantels fixiert wird.

Zu Fig. 18a ist noch anzumerken, dass insbesondere bei einer Miniaturisierung des Einwegmantels die beiden Schnapprinnen 67 auch näher beieinander angeordnet sein können, wobei die zueinander benachbarten Seitenwände 69 der beiden Schnapprinnen 67 sogar ineinander übergehen können.

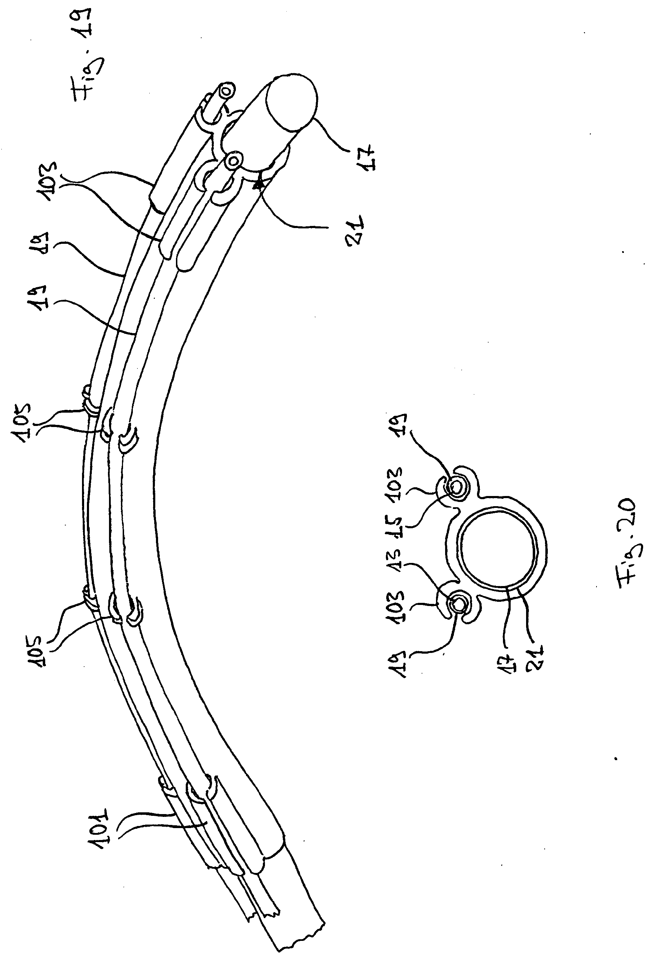

Fig. 19 zeigt in einer schematischen Perspektivansicht eines Einwegmantels mit einem Endoskopkanal 21 zur Aufnahme eines Endoskops 17 sowie mit zwei jeweils mehrteiligen Außenkopplungseinrichtungen zur Ankopplung jeweils eines Arbeitsgeräts 19. Jede Außenkopplungseinrichtung besitzt einen elastischen proximalen Schnapprinnenabschnitt 101, einen elastischen distalen Schnapprinnenabschnitt 103 sowie zwei dazwischen angeordnete elastische Schnappklammern 105, die - bei geradliniger Ausrichtung des Einwegmantels - entlang einer gemeinsamen Längsachse angeordnet sind. Jedes Arbeitsgerät 19 kann in die Schnapprinnenabschnitte 101, 103 und Schnappklammern 105 der zugeordneten Außenkopplungseinrichtung seitlich eingeklippt werden, um das Arbeitsgerät 19 temporär an das Endoskop 17 anzukoppeln. Fig. 20 zeigt eine Frontansicht des distalen Endes der somit hergestellten Anordnung.

Innerhalb der Schnapprinnenabschnitte 101, 103 sind die Arbeitsgeräte 19 kraftschlüssig gehalten, so dass ein axiales Verschieben der Arbeitsgeräte 19 nur entgegen einer vergleichsweise hohen Reibungskraft möglich ist. Dadurch sind die distalen Enden der Arbeitsgeräte 19 bezüglich des distalen Endes des in Fig. 19 gezeigten Einwegmantels und somit bezüglich des distalen Endes des darin aufgenommenen Endoskops 17 axial fixiert und gegen ein unerwünschtes Verschieben gesichert. Innerhalb der Schnappklammern 105 sind die Arbeitsgeräte 19 hingegen lediglich formschlüssig und somit frei beweglich gelagert.

Die erläuterte Befestigung der Arbeitsgeräte 19 innerhalb der Schnapprinnenabschnitte 101, 103 und der Schnappklammern 105 eignet sich besonders gut für den Fall, dass als Arbeitsgeräte 19 Saug-/ Spülschläuche mit Saug-/Spülkanälen 13, 15 eingesetzt werden sollen. In diesem Fall kann nämlich die Gefahr eines unerwünschten Kollabierens, also beispielsweise Einknickens oder Einschnürens des jeweiligen Saug-/Spülkanals 13, 15 bestehen. Diese Gefahr wird bei der Ausführungsform gemäß Fig. 19 und 20 verringert, da die Saug-/ Spülschläuche 19 sowohl am proximalen Ende als auch am distalen Ende des Einwegmantels mittels der Schnapprinnenabschnitte 101, 103, wie erläutert, axial fixiert sind. Da ferner die Saug-/Spülschläuche 19 außerhalb des Krümmungsrädius des Endoskops 17 angeordnet sind (vgl. Fig. 19), führt die Krümmung des Endoskops 17 und somit des gezeigten Einwegmantels letztlich zu einem axialen Dehnen der proximal und distal fixierten Saug-/Spülschläuche 19. Diese Dehnung bewirkt eine Stabilisierung der Kanalwände der Saug-/Spülkanäle 13, 15, so dass einem Kollabieren entgegengewirkt wird.

Auch bei dem in Fig. 20 gezeigten Ausführungsbeispiel können die beiden Schnapprinnenabschnitte 103 - insbesondere bei einer Miniaturisierung des Einwegmantels - noch näher beieinander angeordnet sein. Um mittels der beiden Saug-/ Spülkanäle 13, 15 dennoch einen Fluidstrom herbeiführen zu können, der quer über das distale Ende des Endoskops 17 und eine daran angeordnete Optik verläuft, kann an dem Einwegmantel, an den Schnapprinnenabschnitten 103 oder an den Saug-/ Spülschläuchen 19 wenigstens eine Umlenkeinrichtung, beispielsweise ein Umlenkflügel vorgesehen sein, wie im Zusammenhang mit Fig. 6a bereits erläutert.

Alternativ zu der erläuterten benachbarten Anordnung der beiden Schnapprinnenabschnitte 103 kann auch eine im Wesentlichen diametral gegenüberstehende Anordnung vorgesehen sein, um einen Fluidstrom quer über das distale Ende des Endoskops 17 herbeizuführen.

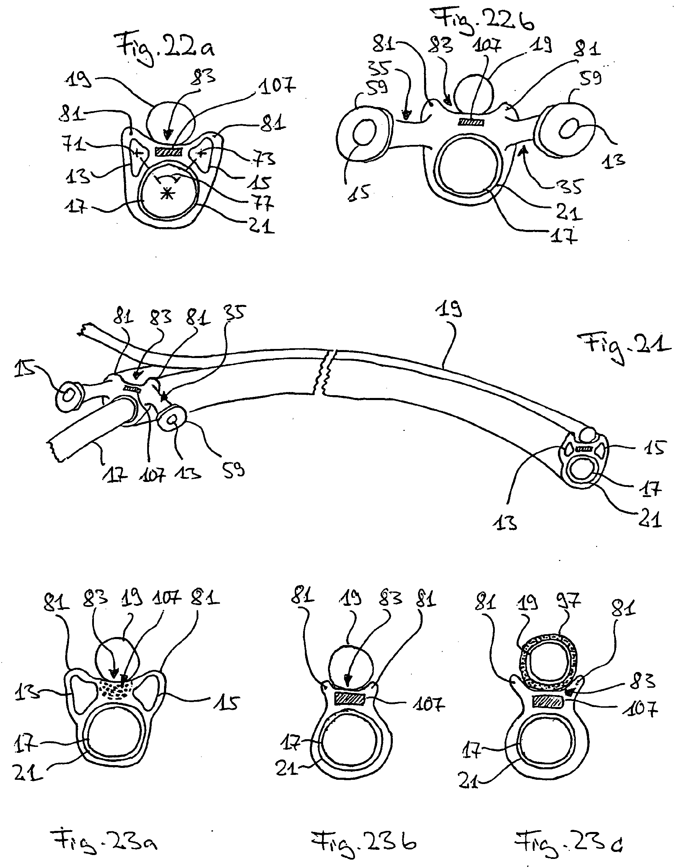

Fig. 21 zeigt in einer schematischen Perspektivansicht eine weitere Ausführungsform eines erfindungsgemäßen Einwegmantels. Fig. 22a und 22b zeigen eine Draufsicht des distalen Endes bzw. des proximalen Endes dieses Einwegmantels.

Der Einwegmantel gemäß Fig. 21, 22a und 22b besitzt einen Endoskopkanal 21 zur Aufnahme eines Endoskops 17 sowie einen Spülkanal 13 und einen Saugkanal 15, deren Längsachsen 71 bzw. 73 bezüglich der Längsachse 75 des Endoskopkanals 21 einen Winkel 77 von etwa 80° relativ zueinander einnehmen (vgl. Fig. 22a).

Als Außenkopplungseinrichtung ist seitens des Einwegmantels eine Magnetkopplungseinrichtung 107 vorgesehen. Diese ist durch ein permanentmagnetisches Band gebildet, das sich entlang der gesamten Länge des Einwegmantels erstreckt, und zwar zwischen dem Spülkanal 13 und dem Saugkanal 15 an der Außenseite des Einwegmantels. Zur Ankopplung an diese Magnetkopplungseinrichtung 107 besteht ein Arbeitsgerät 19 zumindest teilweise aus einem magnetischen Material, beispielsweise einem magnetischen Metall.

Wie insbesondere aus der Frontansicht gemäß Fig. 22a ersichtlich ist, sind die Wandungen des Spülkanals 13 und des Saugkanals 15 als Umfangserhöhungen 81 ausgebildet, die einen konkaven Umfangsabschnitt 83 zur Aufnahme des Arbeitsgeräts 19 einschließen. Entlang des Zentrums dieses Umfangsabschnitts 83 erstreckt sich die Magnetkopplungseinrichtung 107.

Das magnetisch angekoppelte Arbeitsgerät 19 ist innerhalb des Umfangsabschnitts 83 entlang der Längsrichtung des Einwegmantels axial verschiebbar. Aufgrund des flächigen Anliegens an dem Einwegmantel erfordert ein derartiges Verschieben jedoch das Überwinden einer vergleichsweise großen Reibungskraft, so dass gleichzeitig ein unbeabsichtigtes axiales Verschieben des Arbeitsgeräts 19 verhindert wird.

Die Frontansicht des proximalen Endes des Einwegmantels gemäß Fig. 22b zeigt, dass der Spülkanal 13 und der Saugkanal 15 jeweils einen angeformten Kanalfortsatz 35 aufweisen, der an einem Anschlussflansch 59 mündet, der als Verbindungseinrichtung zum Anschluss an eine Pumpe bzw. einen Spülbehälter dient.

Fig. 23a bis 23e zeigen Querschnittsansichten weiterer Ausführungsbeispiele von Einwegmänteln, die einen Endoskopkanal 21 zur Aufnahme eines Endoskops 17 sowie eine integrierte Magnetkopplungseinrichtung 107 zur magnetischen Ankopplung eines Arbeitsgeräts 19 besitzen.

Fig. 23a zeigt ein Ausführungsbeispiel, bei dem die Magnetkopplungseinrichtung 107 durch ein permanentmagnetisches Pulver gebildet ist, das entlang des konkaven Umfangsabschnitts 83 in den Einwegmantel integriert, beispielsweise eingeschmolzen ist.

Fig. 23b zeigt ein Ausführungsbeispiel, bei dem - im Unterschied zu der Ausführungsform gemäß Fig. 23a - lediglich ein Endoskopkanal 21 und ein Magnetband 107 mit umgebenden Umfangserhöhungen 81 vorgesehen sind, jedoch kein Saug-/Spülkanal.

Fig. 23c zeigt einen Einwegmantel wie bei Fig. 23b. Zur Ankopplung eines Arbeitsgeräts 19, das nicht magnetisch ist und demzufolge keine Gegenkopplungseinrichtung für die Magnetkopplungseinrichtung 107 des Einwegmantels besitzt, ist ein Ankopplungsschlauch 97 vorgesehen, in den das Arbeitsgerät 19 eingeführt ist und der magnetisch ist. Somit bildet der Ankopplungsschlauch 97 das erforderliche Magnetgegenelement. Der Ankopplungsschlauch 97 kann beispielsweise aus Kunststoff gebildet sein, in den magnetisches Pulver in einer gleichmäßigen Verteilung entlang des Umfangs integriert ist.

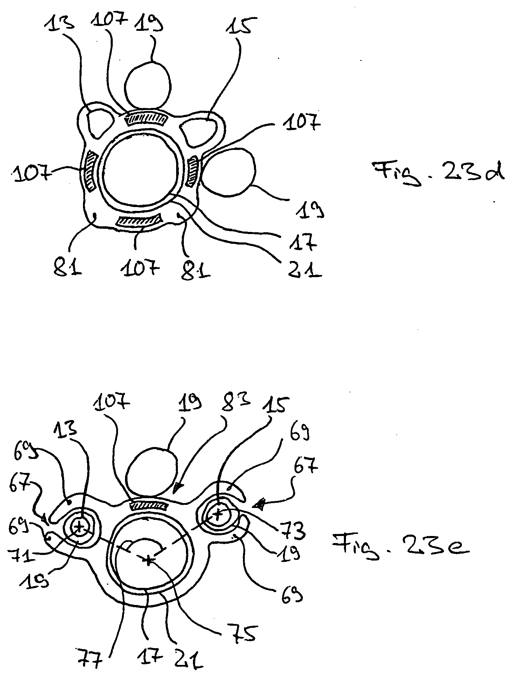

Fig. 23d zeigt ein Ausführungsbeispiel eines Einwegmantels zur Ankopplung mehrerer Arbeitsgeräte 19. Zu diesem Zweck sind vier Magnetkopplungseinrichtungen 107 gleichmäßig über den Umfang verteilt. Diese Magnetkopplungseinrichtungen 107 können beispielsweise durch permanentmagnetische Bänder gebildet sein, die sich entlang der gesamten Länge oder lediglich eines Längsabschnitts des Einwegmantels erstrecken. Zwischen den Magnetkopplungseinrichtungen 107 sind Saug-/Spülkanäle 13, 15 oder Umfangserhöhungen 81 des Einwegmantels vorgesehen.

Fig. 23e zeigt ein Ausführungsbeispiel, bei dem zwei Schnapprinnen 67 an dem Einwegmantel angeformt sind, zwischen denen ein konkaver Umfangsabschnitt 83 ausgebildet und eine Magnetkopplungseinrichtung 107 zur Aufnahme eines magnetischen Arbeitsgeräts 19 angeordnet ist. Die Längsachsen 71, 73 der beiden Schnapprinnen 67 nehmen bezüglich der Längsachse 75 des Endoskopkanals 21 einen Winkel 77 von etwa 120° ein. Als Arbeitsgeräte 19 können beispielsweise Saug-/Spülschläuche mit jeweils einem Saug-/ Spülkanal 13 bzw. 15 in die Schnapprinnen 67 eingeklippt werden. Falls hierbei ein Kraftschluss zwischen den Seitenwänden 69 der Schnapprinnen 67 einerseits und dem jeweiligen Saug-/ Spülschlauch 19 andererseits bewerkstelligt wird, kann diese Anordnung in vorteilhafter Weise zur Verringerung der Gefahr eines Kollabierens der Saug-/Spülkanäle 13, 15 beitragen, wie im Zusammenhang mit Fig. 19 und 20 bereits erläutert.

Der in Fig. 23e gezeigte Winkel 77 kann auch derart geringer gewählt werden, dass die beiden Schnapprinnen 67 eine Aufnahmerinne 83 zur im Wesentlichen formschlüssigen Aufnahme des magnetischen Arbeitsgeräts 19 bilden. Beispielsweise kann - je nach Durchmesser des Einwegmantels, nach Durchmesser der Schnapprinnen 67 und nach Umfang des magnetischen Arbeitsgeräts 19 - ein Winkel 77 von ca. 30°, 45°, 60° oder 90° gewählt werden.

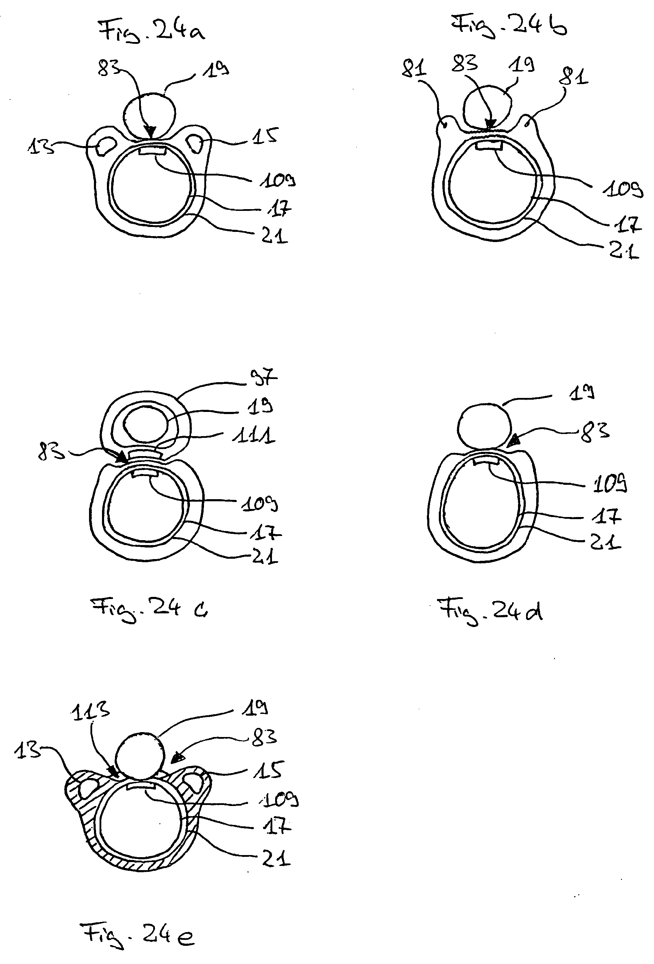

Fig. 24a bis 24e zeigen Querschnittsansichten von Ausführungsformen eines Einwegmantels, der eine magnetische Ankopplung eines Arbeitsgeräts 19 an ein Endoskop 17 ermöglicht, ohne dass der Einwegmantel zu diesem Zweck eine eigene Magnetkopplungseinrichtung aufweisen muss. Stattdessen ist das Endoskop 17 mit einer eigenen Magnetkopplungseinrichtung 109 versehen, die sich beispielsweise an der Außenseite des Endoskops 17 entlang im Wesentlichen der gesamten Länge des Endoskops 17 erstreckt und beispielsweise durch einen Permanentmagneten gebildet ist.

Fig. 24a zeigt ein Ausführungsbeispiel eines solchen Einwegmantels, der einen Endoskopkanal 17 sowie einen Spülkanal 13 und einen Saugkanal 15 besitzt, die einen konkaven Umfangsabschnitt 83 zur Aufnahme eines Arbeitsgeräts 19 umgeben. Um ein magnetisches Zusammenwirken der Magnetkopplungseinrichtung 109 des Endoskops 17 mit dem Arbeitsgerät 19 zu ermöglichen bzw. zu begünstigen, ist der genannte Umfangsabschnitt 83 als Mantelverdünnung des Einwegmantels bzw. des Endoskopkanals 21 ausgebildet, der bezüglich des restlichen Umfangs des Einwegmantels eine deutlich verminderte Wandstärke besitzt.

Fig. 24b zeigt ein ähnliches Ausführungsbeispiel, wobei der genannte Umfangsabschnitt 83 nicht nur besonders dünnwandig, sondern außerdem elastisch ausgebildet ist, um eine Anpassung des Endoskopkanals 21 an Endoskope 17 unterschiedlichen Durchmessers zu ermöglichen. Außerdem sind anstelle des Spülkanals 13 und Saugkanals 15 gemäß Fig. 24a lediglich zwei den Umfangsabschnitt 83 umgebende Umfangserhöhungen 81 vorgesehen.

Fig. 24c zeigt ein Ausführungsbeispiel, bei dem der Einwegmantel einen Endoskopkanal 21 aufweist, dessen Wandstärke entlang eines Umfangsabschnitts 83 deutlich verringert ist. Zur Ankopplung eines nicht magnetischen Arbeitsgeräts 19 an ein Endoskop 17, das sich innerhalb des Endoskopkanals 21 befindet und eine Magnetkopplungseinrichtung 109 aufweist, ist ein Ankopplungsschlauch 97 vorgesehen. Dieser nimmt das Arbeitsgerät 19 auf und besitzt eine Magnetgegeneinrichtung 111, beispielsweise in Form eines flexiblen Metallbands an der Außenseite des Ankopplungsschlauchs 97.

Fig. 24d zeigt eine Ausführungsform eines Einwegmantels mit einem Endoskopkanal 21 und einem Umfangsabschnitt 83 verminderter Wandstärke, ähnlich dem Ausführungsbeispiel gemäß Fig. 24c. Allerdings ist der Querschnitt dieses Endoskopkanals 21 nicht kreisrund und somit rotationssymmetrisch, sondern oval. Sofern das in diesem Endoskopkanal 21 befindliche Endoskop 17 formschlüssig zu dieser Querschnittsform ausgebildet ist, ist gewährleistet, dass sich das Endoskop 17 mit der daran angeordneten Magnetkopplungseinrichtung 109 nicht unbeabsichtigt bezüglich des Umfangsabschnitts 83 zur Ankopplung des Arbeitsgeräts 19 verdreht.

Fig. 24e zeigt ein Ausführungsbeispiel, das jenem gemäß Fig. 24a entspricht. Allerdings ist als Kopplungsübertragungsabschnitt des Einwegmantels hier keine Mantelverdünnung entlang des konkaven Umfangsabschnitts 83 vorgesehen, sondern eine Umfangsöffnung 113, die ein unmittelbares Anliegen des Arbeitsgeräts 19 an dem Endoskop 17 und somit an dessen Magnetkopplungseinrichtung 109 ermöglicht.

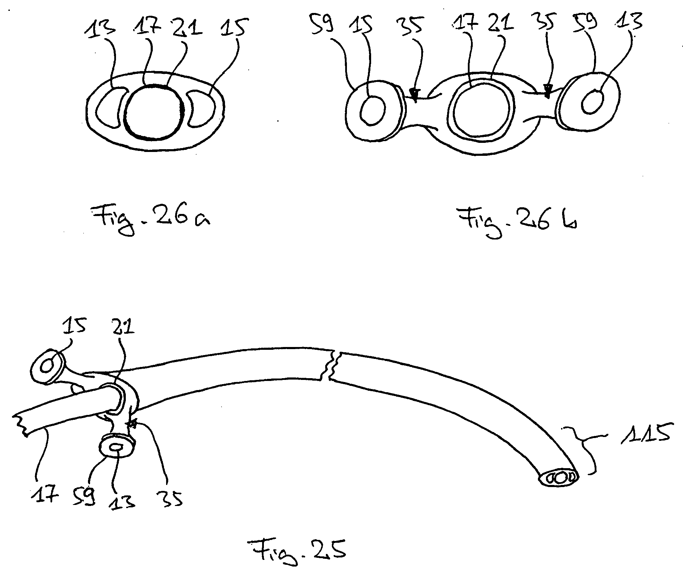

Fig. 25 zeigt in einer schematischen Perspektivansicht eine Ausführungsform eines erfindungsgemäßen Einwegmantels, der dazu dient, ein Kompaktendoskop 17 mit einem Spülkanal 13 und einem Saugkanal 15 zu versehen. Fig. 26a und 26b zeigen jeweils eine Frontansicht des distalen Endes bzw. des proximalen Endes dieses Einwegmantels.

Dieser Einwegmantel besitzt einen zentralen Endoskopkanal 21 zur Aufnahme des Endoskops 17 sowie zwei seitliche, einander gegenüberstehende Saug-/Spülkanäle 13, 15. Die Saug-/Spülkanäle 13, 15 sind am proximalen Ende des Einwegmantels als Fortsätze 35 mit einem jeweiligen Anschlussflansch 59 ausgebildet, wie im Zusammenhang mit Fig. 7 bis 10 bereits erläutert (vgl. Fig. 26b).

Am distalen Ende des Einwegmantels ist der Endoskopkanal 21 dergestalt verengt, dass er das aufgenommene Endoskop 17 kraftschlüssig umgibt (vgl. Fig. 26a). Eine derartige Verengung ist entlang eines distalen Verengungsabschnitts 115 des Einwegmantels vorgesehen. Diese Verengung des Endoskopkanals 21 dient als Fixiereinrichtung, durch die die Lage des distalen Endes des Endoskops 17 relativ zu dem Einwegmantel fixiert wird. Für eine axiale Bewegung des Endoskops 17 muss nämlich der erläuterte Reibungskraftschluss überwunden werden, was nur durch einen vergleichsweise hohen Kraftaufwand möglich ist und deswegen nicht leicht unbeabsichtigt geschieht.

Im Übrigen besitzt auch der Einwegmantel gemäß Fig. 25, 26a und 26b den erläuterten Vorteil, dass ein Kompaktendoskop 17 im Zusammenhang mit einem Spülkanal 13 und einem Saugkanal 15 verwendet werden kann, ohne dass die Saug-/Spülkanäle 13, 15 an dem Endoskop 17 selbst ausgebildet sein müssen und sich bezüglich einer hygienisch einwandfreien Reinigung dieses Endoskops 17 als problematisch erweisen könnten. Statt dessen wird das Endoskop 17 für einen einmaligen Gebrauch in den Endoskopkanal 21 des Einwegmantels eingeführt. Nach dem Gebrauch wird der Einwegmantel mit den Saug-/Spülkanälen 13, 15 abgenommen und entsorgt, und das Endoskop 17 kann auf übliche Weise gereinigt werden.

### Bezugszeichenliste

- 11: Endoskop-/Arbeitskanal
- 13: Spülkanal
- 15: Saugkanal
- 17: Endoskop
- 19: Arbeitsgerät
- 21: Endoskopkanal
- 23: Arbeitskanal
- 25: Führungssteg
- 27: Spül-/Saugkanal
- 29: Druckluftkanal
- 31: Führungssteg
- 33: Umlenkeinrichtung
- 35: Spül-/Saugkanalfortsatz
- 37: Fixierschraube
- 39: Gewindeöffnung
- 41: Verschlussschraube
- 43: Mehrweg-Rastverschluss
- 45: Grundelement
- 47: Verschlusselement
- 49: Membran
- 51: Verschlusspfropfen
- 53: Rastelement
- 55: Gegenelement
- 57: Membran
- 59: Anschlussflansch
- 61: Verbindungsende
- 63: Außengewinde
- 65: Abschlussverbreiterung
- 67: Schnapprinne
- 69: Seitenwand
- 71: Längsachse des Spülkanals
- 73: Längsachse des Saugkanals
- 75: Längsachse des Endoskopkanals
- 77: Anordnungswinkel
- 79: Längsabschnitt des Einwegmantels
- 81: Umfangserhöhung
- 83: konkaver Umfangsabschnitt
- 85: Lichtleiter
- 87: Aufnahme optik
- 89: elastischer Umfangsabschnitt
- 91: elastischer Umfangsabschnitt
- 93: Nutverbindungseinrichtung
- 95: Schienenverbindungseinrichtung
- 97: Ankopplungsschlauch
- 99: Magnetkopplungseinrichtung
- 101: proximaler Schnapprinnenabschnitt
- 103: distaler Schnapprinnenabschnitt
- 105: Schnappklammer
- 107: Magnetkopplungseinrichtung
- 109: Magnetkopplungseinrichtung
- 111: Magnetgegeneinrichtung
- 113: Umfangsöffnung
- 115: Verengungsabschnitt

## Patentansprüche

1. Einwegmantel für einen einmaligen Gebrauch, zumindest mit einem Endoskopkanal (11, 21) zur Aufnahme eines Endoskops (17), wobei wenigstens ein von dem Endoskopkanal (11, 21) separater Spülkanal (13, 27) zur Übertragung einer Flüssigkeit oder eines Gases zum distalen Ende des Einwegmantels vorgesehen ist,
**dadurch gekennzeichnet,**
**dass** wenigstens eine Fixiereinrichtung vorgesehen ist, durch die die Lage des distalen Endes des Endoskops relativ zu dem Einwegmantel fixierbar ist, wobei die Fixiereinrichtung einen Einschnürbund am distalen Ende des Endosköpkanals aufweist, der für einen Kraftschluss mit dem Endoskop (17) und somit für eine erhöhte Reibung sorgt, so dass ein Verschieben des in dem Einschnürbund gefangenen Endoskops erschwert ist, und
**dass** am distalen Ende des Einwegmantels im Bereich einer Austrittsöffnung des Spülkanals (13, 27) eine Umlenkeinrichtung (33) zum Umlenken einer durch den Spülkanal (13, 27) übertragenen Spülflüssigkeit oder eines Spülgases vorgesehen ist.

2. Einwegmantel nach Anspruch 1,
**dadurch gekennzeichnet,**
**dass** der Endoskopkanal als Endoskop-/Arbeitskanal (11) zur zusätzlichen Aufnahme eines Arbeitsgeräts (19) ausgebildet ist, wobei der Endoskop-/Arbeitskanal (11) insbesondere einen im Wesentlichen ovalen Innenquerschnitt aufweist und/oder wenigstens einen, vorzugsweise zwei gegenüberliegende Führungsstege (25, 31) zum Führen des Endoskops (17) und des Arbeitsgeräts (19) aufweist.

3. Einwegmantel nach Anspruch 1,
**dadurch gekennzeichnet,**
**dass** der Endoskopkanal (21) zur Aufnahme lediglich des Endoskops (17) ausgebildet ist und dass wenigstens ein vom Endoskopkanal (21) separater Arbeitskanal (23) zur Aufnahme eines Arbeitsgeräts (19) vorgesehen ist,
wobei der Endoskopkanal (21) und/oder der Arbeitskanal (23) insbesondere jeweils einen im Wesentlichen kreisrunden Innenquerschnitt aufweist.

4. Einwegmantel nach einem der vorhergehenden Ansprüche,
**dadurch gekennzeichnet,**
**dass** der Einwegmantel wenigstens eine Außenkopplungseinrichtung (67, 93, 99, 101, 103, 105, 107) für eine Ankopplung eines Arbeitsgeräts (19) an die Außenseite des Einwegmantels aufweist.

5. Einwegmantel nach Anspruch 4,
**dadurch gekennzeichnet,**
**dass** die Außenkopplungseinrichtung eine Schnappverbindungseinrichtung (67, 101, 103, 105), eine Magnetkopplungseinrichtung (99, 107), eine Klettverschlusseinrichtung, eine Klebeverbindungseinrichtung und/oder eine Schienen-/Nutverbindungseinrichtung (93) aufweist,
wobei die Schnappverbindungseinrichtung vorzugsweise wenigstens eine elastische Schnapprinne (67, 101, 103) oder eine elastische Schnappklammer (105) zum Umgreifen des Arbeitsgeräts (19) aufweist, und/oder
wobei die Magnetkopplungseinrichtung (99, 107) vorzugsweise
- einen Permanentmagneten aufweist,
- sich in Längsrichtung des Einwegmantels durchgehend oder lediglich entlang eines oder mehrerer Längsabschnitte erstreckt,
- an einem distalen Ende des Einwegmantels vorgesehen ist,
- innerhalb des Einwegmantels pulverförmig verteilt ist, und/oder
- als austauschbare Mehrwegeinheit ausgebildet ist.

6. Einwegmantel nach einem der Ansprüche 4 und 5,
**dadurch gekennzeichnet,**
**dass** die Außenkopplungseinrichtung (67, 93, 99, 101, 103, 105, 107) lediglich entlang eines oder mehrerer Umfangsabschnitte des Einwegmantels vorgesehen ist, und/oder
**dass** das Arbeitsgerät (19) wenigstens eine mit der Außenkopplungseinrichtung (67, 93, 99, 101, 103, 105, 107) zusammenwirkende Gegenkopplungseinrichtung (95, 111) aufweist, und/oder dass das Arbeitsgerät (19) relativ zu der Außenkopplungseinrichtung (67, 93, 99, 101, 103, 105, 107) in Längsrichtung des Einwegmantels bewegbar ist.

7. Einwegmantel nach einem der Ansprüche 4 bis 6,
**dadurch gekennzeichnet,**
**dass** eine Fixiereinrichtung vorgesehen ist, durch die die Lage des Arbeitsgeräts (19) relativ zu der Längsrichtung des Einwegmantels fixierbar ist,
wobei die Fixiereinrichtung vorzugsweise
- am distalen Ende des Einwegmantels vorgesehen ist,
- einen Permanentmagneten (99), einen Elektromagneten oder eine Einhakeinrichtung am distalen Ende des Einwegmantels aufweist,
- einen Permanentmagneten (99) an einem Längsabschnitt des Einwegmantels aufweist, und/oder
- eine arretierbare Fixierklammer am proximalen Ende des Einwegmantels aufweist.

8. Einwegmantel nach einem der Ansprüche 4 bis 7,
**dadurch gekennzeichnet,**
**dass** der Einwegmantel zwei die Außenkopplungseinrichtung seitlich umgebende Umfangserhöhungen (81) aufweist, die einen im Querschnitt konkaven Umfangsabschnitt (83) zur Aufnahme des Arbeitsgeräts (19) einschließen, und/oder
**dass** der Einwegmantel mehrere, insbesondere zwei oder drei Außenkopplungseinrichtungen (67, 93, 99, 101, 103, 105, 107) für eine Ankopplung einer entsprechenden Anzahl von Arbeitsgeräten (19) aufweist, und/oder
**dass** wenigstens eine Außenkopplungseinrichtung (67, 99) für eine derartige Ankopplung eines Arbeitsgeräts (19) ausgebildet ist, dass das distale Ende des Arbeitsgeräts an einem Längsabschnitt zwischen dem distalen und dem proximalen Ende des Einwegmantels angeordnet, insbesondere fixiert ist.

9. Einwegmantel nach einem der Ansprüche 4 bis 8,
**dadurch gekennzeichnet,**
**dass** ferner ein Ankopplungsschlauch (97) vorgesehen ist, der zur Ankopplung an den Einwegmantel wenigstens eine mit der Außenkopplungseinrichtung (67, 93, 107) zusammenwirkende Gegenkopplungseinrichtung (95, 111) aufweist,
wobei der Ankopplungsschlauch (97) insbesondere als ein Einwegteil vorgesehen und/ oder zur Aufnahme des Arbeitsgeräts (19) ausgebildet ist.

10. Einwegmantel nach einem der Ansprüche 4 bis 9,
**dadurch gekennzeichnet,**
**dass** als Arbeitsgerät ein Saug-/ Spülschlauch (19) mit wenigstens einem Saug-/Spülkanal (13, 15) vorgesehen ist, wobei der Saug-/ Spülschlauch vorzugsweise
- durch eine distale Fixiereinrichtung (103) dergestalt bezüglich der Längsrichtung des Einwegmantels fixierbar ist, dass das distale Ende des Saug-/Spülkanals einen vorbestimmten Abstand zu dem distalen Ende des Einwegmantels oder des darin aufgenommenen Endoskops (17) besitzt, und/oder
- durch eine proximale (101) und eine distale (103) Außenkopplungseinrichtung dergestalt bezüglich der Längsrichtung des Einwegmantels fixierbar ist, dass bei einer Krümmung des Einwegmantels der Saug-/Spülschlauch gespannt oder gedehnt wird, und/oder
- in Längsrichtung elastisch dehnbar ist, und/oder
- am distalen Ende eine Umlenkeinrichtung zum Umlenken einer Spülflüssigkeit oder eines Spülgases aufweist.

11. Einwegmantel nach einem der Ansprüche 4 bis 10,
**dadurch gekennzeichnet,**
**dass** der Einwegmantel wenigstens eine Magnetkopplungseinrichtung (107) für eine Ankopplung eines Arbeitsgeräts (19) an die Außenseite des Einwegmantels sowie zwei die Magnetkopplungseinrichtung umgebende Außenkopplungseinrichtungen (67) für eine Ankopplung jeweils eines Saug-/Spülschlauchs (19) aufweist, und/oder
**dass** der Endoskopkanal (21) zur Aufnahme lediglich des Endoskops (17) ausgebildet ist.

12. Einwegmantel nach einem der vorhergehenden Ansprüche,
**dadurch gekennzeichnet,**
**dass** wenigstens ein den Endoskopkanal (21) begrenzender Umfangsabschnitt des Einwegmantels als ein Kopplungsübertragungsabschnitt (83, 113) ausgebildet ist, der für eine Außenkopplung eines Arbeitsgeräts (19) an das in dem Einwegmantel befindliche Endoskop (17) ein Zusammenwirken einer Magnethalteeinrichtung (109) des Endoskops mit einer Magnetgegeneinrichtung (111) des Arbeitsgeräts ermöglicht.

13. Einwegmantel nach Anspruch 12,
**dadurch gekennzeichnet,**
**dass** der Kopplungsübertragungsabschnitt des Einwegmantels eine Mantelverdünnung (83) oder eine Umfangsöffnung (113) aufweist, durch die die Magnethalteeinrichtung (109) und die Magnetgegeneinrichtung (111) zusammenwirken, und/oder dass entlang der Längsrichtung des Einwegmantels ein durchgehender oder mehrere separate Kopplungsübertragungsabschnitte (83, 113) vorgesehen sind, und/oder
**dass** ein Kopplungsübertragungsabschnitt (83, 113) an einem distalen Ende des Einwegmantels vorgesehen ist.

14. Einwegmantel nach einem der Ansprüche 12 und 13,
**dadurch gekennzeichnet,**
**dass** als Arbeitsgerät ein Saug-/ Spülschlauch (19) mit wenigstens einem Saug-/ Spülkanal (13, 15) vorgesehen ist, und/oder dass der Einwegmantel zwei Umfangserhöhungen (81) aufweist, die den Kopplungsübertragungsabschnitt (83, 113) seitlich umgeben und die einen im Querschnitt konkaven Umfangsabschnitt zur Aufnahme des Arbeitsgeräts einschließen, und/oder dass ferner ein Ankopplungsschlauch (97) vorgesehen ist, der zur Aufnahme des Arbeitsgeräts (19) ausgebildet ist und die Magnetgegeneinrichtung (111) aufweist, wobei der Ankopplungsschlauch (97) insbesondere als ein Einwegteil vorgesehen ist.

15. Einwegmantel nach einem der Ansprüche 4 bis 14,
**dadurch gekennzeichnet,**
**dass** der Einwegmantel zwei Saug-/Spülkanäle aufweist (13, 15), die relativ zu dem Endoskopkanal (21) dergestalt angeordnet sind, dass der Einwegmantel an seiner Außenseite zwischen den Saug-/ Spülkanälen einen im Querschnitt konkaven Umfangsabschnitt (83) zur Aufnahme des Arbeitsgeräts (19) aufweist.

16. Einwegmantel nach Anspruch 15,
**dadurch gekennzeichnet,**
**dass** eine Außenkopplungseinrichtung (67, 93, 99, 101, 103, 105, 107) oder ein Kopplungsübertragungsabschnitt (83, 113) an dem konkaven Umfangsabschnitt vorgesehen ist, und/oder dass der Einwegmantel zumindest entlang des konkaven Umfangsabschnitts (83) elastisch ausgebildet ist, und/oder dass die Längsachsen (71, 73) der Saug-/Spülkanäle (13, 15) bezüglich der Längsachse (75) des Endoskopkanals (21) im Querschnitt einen Winkel zwischen 30° und 120°, insbesondere einen Winkel von etwa 45°, 60° oder 90° relativ zueinander einnehmen.

17. Einwegmantel nach einem der vorhergehenden Ansprüche 2 - 16, **dadurch gekennzeichnet,**
**dass** der zur Aufnahme eines Arbeitsgeräts (19) vorgesehene Kanal (11, 23) eine Versteifung aufweist, die sich insbesondere entlang eines bei Krümmung des Einwegmantels krümmungsaußenseitig verlaufenden Umfangsabschnitts und/oder entlang eines Längsabschnitts des Einwegmantels erstreckt, und/oder dass ein oder zwei zusätzliche Arbeitskanäle vorgesehen sind, und/oder
**dass** wenigstens eine Fixiereinrichtung (37) vorgesehen ist, durch die die Lage des Arbeitsgeräts (19) relativ zu dem Einwegmantel temporär fixierbar ist, wobei die Fixiereinrichtung vorzugsweise eine Fixierschraube (37) aufweist.

18. Einwegmantel nach einem der vorhergehenden Ansprüche,
**dadurch gekennzeichnet,**
**dass** das proximale Ende des Spülkanals (13) eine Verbindungseinrichtung (59, 63) zum Anschluss an eine Pumpe oder einen Spülbehälter aufweist, wobei die Verbindungseinrichtung insbesondere einen flexiblen Schlauch, einen Anschlussflansch (59) und/oder ein Gewinde (63) aufweist.

19. Einwegmantel nach einem der vorhergehenden Ansprüche,
**dadurch gekennzeichnet,**
**dass** der Spülkanal wenigstens eine Verschlusseinrichtung (39, 41, 51) zum temporären Verschließen des Spülkanals oder wenigstens eine Aufnahmeeinrichtung zur Aufnahme einer Mehrweg-Verschlusseinrichtung (43) aufweist, wobei vorzugsweise eine Trenneinrichtung, insbesondere eine flexible Membran (49) zur Trennung der Mehrweg-Verschlusseinrichtung (43) vom Spülkanal (13) vorgesehen ist.

20. Einwegmantel nach einem der vorhergehenden Ansprüche,
**dadurch gekennzeichnet,**
**dass** wenigstens ein Saugkanal (15) zum Absaugen von Flüssigkeit oder Gas am distalen Ende des Einwegmantels vorgesehen ist, wobei das proximale Ende des Saugkanals (15) vorzugsweise eine Verbindungseinrichtung (59, 63) zum Anschluss an eine Pumpe aufweist, wobei die Verbindungseinrichtung insbesondere einen flexiblen Schlauch, einen Anschlussflansch (59) und/oder ein Gewinde (63) aufweist.

21. Einwegmantel nach Anspruch 20,
**dadurch gekennzeichnet,**
**dass** der Saugkanal (15) wenigstens eine Verschlusseinrichtung (39, 41, 51) zum temporären Verschließen des Saugkanals oder wenigstens eine Aufnahmeeinrichtung zur Aufnahme einer Mehrweg-Verschlusseinrichtung (43) aufweist, wobei vorzugsweise eine Trenneinrichtung, insbesondere eine flexible Membran (49) zur Trennung der Mehrweg-Verschlusseinrichtung (43) vom Saugkanal (15) vorgesehen ist, und/oder
**dass** die distale Öffnung des Spülkanals (13) und die distale Öffnung des Saugkanals (15) bezüglich der distalen Öffnung des Endoskopkanals (11, 21) gegenüber voneinander angeordnet sind.

22. Einwegmantel nach einem der vorhergehenden Ansprüche,
**dadurch gekennzeichnet,**
**dass** der Einwegmantel einen im Wesentlichen kreisrunden, ovalen oder einen dem Querschnitt des menschlichen Nasengangs entsprechenden Außenquerschnitt aufweist, und/oder dass der Einwegmantel aus einem flexiblen, insbesondere einem elastischen Material gebildet ist, vorzugsweise aus einem Kunststoff, und/oder
**dass** der Einwegmantel hinsichtlich seines gesamten Umfangs oder hinsichtlich eines Umfangsabschnitts (89, 91) elastisch ausgebildet ist, und zwar entlang eines Längsabschnitts oder der gesamten Länge des Einwegmantels.

23. Einwegmantel nach einem der vorhergehenden Ansprüche,
**dadurch gekennzeichnet,**
**dass** der Endoskopkanal (21), der Arbeitskanal (22) und/oder der Endoskop-/Arbeitskanal (11) entlang eines oder mehrerer Längsabschnitte des Einwegmantels seitlich geöffnet ist, und/oder dass der Einwegmantel aus einem transparenten Material gebildet ist, und/oder
**dass** zumindest der Umfangsbereich des Einwegmantels, vorzugsweise der gesamte Einwegmantel einstückig ausgebildet ist, und/oder
**dass** die in dem Einwegmantel enthaltenen Kanäle (11, 13, 15, 21, 23) im Wesentlichen bündig zueinander am distalen Ende des Einwegmantels münden, und/oder
**dass** die Kanäle (11, 13, 15, 21, 23) innerhalb des Einwegmantels im Wesentlichen parallel zueinander verlaufen.

24. Einwegmantel nach einem der vorhergehenden Ansprüche,
**dadurch gekennzeichnet,**
**dass** zum Verhindern eines vollständigen Eindringens des Einwegmantels in den Körper des Patienten eine Abschlussverbreiterung (65) am proximalen Ende des Einwegmantels vorgesehen ist, und/oder
**dass** der Einwegmantel eine Länge zwischen 50 und 130 cm aufweist, und/oder
**dass** es sich bei dem Endoskop (17) um ein Kompaktendoskop ohne eigenen Arbeitskanal und insbesondere ohne eigenen Spül- oder Saugkanal handelt, und/oder
**dass** das Endoskop (17) wenigstens einen Licht-/Bildübertragungskanal (85, 87) aufweist, und/oder
**dass** das Endoskop (17) wenigstens einen Bowdenzug aufweist.

## Claims

1. A disposable sheath for single use, at least having an endoscope passage (11, 21) for receiving an endoscope (17), wherein at least one flushing passage (13, 27) separate from the endoscope passage (11, 21) is provided for the transfer of a liquid or of a gas to the distal end of the disposable sheath,
**characterized in that**
at least one fixing device is provided by which the position of the distal end of the endoscope can be fixed relative to the disposable sheath, with the fixing device having a constricting collar at the distal end of the endoscope passage which provides for a frictional connection to the endoscope (17) and thus for an increased friction so that a displacement of the endoscope captured in the constricting collar is made more difficult; and
**in that** a deflection device (33) for deflecting a flushing liquid or a flushing gas transferred through the flushing passage (13, 27) is provided at the distal end of the disposable sheath in the region of an outlet opening of the flushing passage (13, 27).

2. A disposable sheath in accordance with claim 1,
**characterized in that**
the endoscope passage is configured as an endoscope/working passage (11) for the additional reception of an implement (19),
with the endoscope/working passage (11) in particular having a substantially oval inner cross-section and/or at least one guide web, preferably two oppositely disposed guide webs (25, 31), for guiding the endoscope (17) and the implement (19).

3. A disposable sheath in accordance with claim 1,
**characterized in that**
the endoscope passage (21) is configured for receiving only the endoscope (17); and **in that** at least one working passage (23) separate from the endoscope passage (21) is provided for receiving an implement (19),
with the endoscope passage (21) and/or the working passage (23) in particular each having a substantially circular inner cross-section.

4. A disposable sheath in accordance with any one of the preceding claims,
**characterized in that**
the disposable sheath has at least one external coupling device (67, 93, 99, 101, 103, 105, 107) for a coupling of an implement (19) to the outer side of the disposable sheath.

5. A disposable sheath in accordance with claim 4,
**characterized in that**
the external coupling device has a snap-in connection device (67, 101, 103, 105), a magnetic coupling device (99, 107), a hook and loop fastening device, an adhesive connection device and/or a rail/groove connection device (93),
with the snap-in connection device preferably having at least one elastic snap-in channel (67, 101, 103) or an elastic snap-in clamp (105) for engaging around the implement (19); and/or
with the magnet coupling device (99, 107) preferably
- having a permanent magnet;
- extending continuously in the longitudinal direction of the disposable sheath or only along one or more longitudinal sections;
- being provided at a distal end of the disposable sheath;
- being distributed in powder form within the disposable sheath; and/or
- being configured as a replaceable reusable unit.

6. A disposable sheath in accordance with one of the claims 4 and 5, **characterized in that**
the external coupling device (67, 93, 99, 101, 103, 105, 107) is only provided along one or more peripheral sections of the disposable sheath; and/or
**in that** the implement (19) has at least one mating coupling device (95, 111) cooperating with the external coupling device (67, 93, 99, 101, 103, 105, 107); and/or
**in that** the implement (19) is movable in the longitudinal direction of the disposable sheath relative to the external coupling device (67, 93, 99, 101, 103, 105, 107).

7. A disposable sheath in accordance with any one of the claims 4 to 6, **characterized in that**
a fixing device is provided by which the position of the implement (19) is fixable relative to the longitudinal direction of the disposable sheath,
with the fixing device preferably
- being provided at the distal end of the disposable sheath;
- having a permanent magnet (99), an electromagnet or a hook-in device at the distal end of the disposable sheath;
- having a permanent magnet (99) at a longitudinal section of the disposable sheath; and/or
- having a lockable fixing clamp at the proximal end of the disposable sheath.

8. A disposable sheath in accordance with any one of the claims 4 to 7, **characterized in that**
the disposable sheath has two peripheral elevated portions (81) which laterally surround the external coupling device and which enclose a peripheral section (83) concave in cross-section for receiving the implement (19); and/or
**in that** the disposable sheath has a plurality of external coupling devices (67, 93, 99, 101, 103, 105, 107), in particular two or more external coupling devices, for a coupling of a corresponding number of pieces of working equipment (19); and/or
**in that** at least one external coupling device (67, 99) is configured for such a coupling of an implement (19) that the distal end of the implement is arranged, in particular fixed, at a longitudinal section between the distal end and the proximal end of the disposable sheath.

9. A disposable sheath in accordance with any one of the claims 4 to 8, **characterized in that**
a coupling hose (97) is furthermore provided which has a mating coupling device (95, 111) cooperating with the external coupling device (67, 93, 107) for the coupling to the disposable sheath,
with the coupling hose (97) in particular being provided as a disposable part and/or being configured for receiving the implement (19).

10. A disposable sheath in accordance with any one of the claims 4 to 9, **characterized in that**
a suction/flushing hose (19) having at least one suction/flushing passage (13, 15) is provided as the implement, with the suction/flushing hose preferably
- being fixable by a distal fixing device (103) with respect to the longitudinal direction of the disposable sheath such that the distal end of the suction/flushing passage has a predefined spacing from the distal end of the disposable sheath or of the endoscope (17) received therein; and/or
- being fixable by a proximal (101) and a distal (103) external coupling device with respect to the longitudinal direction of the disposable sheath such that the suction/flushing hose is tensioned or stretched on a curvature of the disposable sheath; and/or
- being elastically stretchable in the longitudinal direction; and/or
- having a deflection device at the distal end for the deflection of a flushing liquid or of a flushing gas.

11. A disposable sheath in accordance with any one of the claims 4 to 10,
**characterized in that**
the disposable sheath has at least one magnetic coupling device (107) for a coupling of an implement (19) to the outer side of the disposable sheath and two external coupling devices (67) surrounding the magnetic coupling device for a coupling of a respective suction/flushing hose (19); and/or
**in that** the endoscopic passage (21) is configured for receiving only the endoscope (17).

12. A disposable sheath in accordance with any one of the preceding claims,
**characterized in that**
at least one peripheral section of the disposable sheath bounding the endoscope passage (21) is configured as a coupling transmission section (83, 113) which allows a cooperation of a magnetic holding device (109) of the endoscope with a mating magnetic device (111) of the implement for an external coupling of an implement (19) to the endoscope (17) located in the disposable sheath.

13. A disposable sheath in accordance with claim 12,
**characterized in that**
the coupling transmission section of the disposable sheath has a sheath thinning portion (83) or a peripheral opening (113) through which the magnetic holding device (109) and the mating magnetic device (111) cooperate; and/or
**in that** a continuous coupling transmission section, or a plurality of separate coupling transmission sections (83, 113), is/are provided along the longitudinal direction of the disposable sheath; and/or
**in that** a coupling transmission section (83, 113) is provided at a distal end of the disposable sheath.

14. A disposable sheath in accordance with one of the claims 12 and 13, **characterized in that**
a suction/flushing hose (19) having at least one suction/flushing passage (13, 15) is provided as the implement; and/or
**in that** the disposable sheath has two peripheral elevated portions (81) which laterally surround the coupling transmission section (83, 113) and which enclose a peripheral section concave in cross-section for receiving the implement; and/or
**in that** a coupling hose (97) is furthermore provided which is configured for receiving the implement (19) and comprises the mating magnetic device (111), with the coupling hose (97) in particular being provided as a disposable part.

15. A disposable sheath in accordance with any one of the claims 4 to 14,
**characterized in that**
the disposable sheath has two suction/flushing passages (13, 15) which are arranged relative to the endoscope passage (21) such that the disposable sheath has a peripheral section (83) concave in cross-section at its outer side between the suction/flushing passages to receive the implement (19).

16. A disposable sheath in accordance with claim 15, **characterized in that**
an external coupling device (67, 93, 99, 101, 103, 105, 107) or a coupling transmission section (83, 113) is provided at the concave peripheral section; and/or
**in that** the disposable sheath is configured as elastic at least along the concave peripheral section (83); and/or
**in that** the longitudinal axes (71, 73) of the suction/flushing passages (13, 15) adopt an angle relative to one another with respect to the longitudinal axis (75) of the endoscope passage (21) in cross-section of between 30° and 120°, in particular an angle of approximately 45°, 60° or 90°.

17. A disposable sheath in accordance with any one of the preceding claims 2 to 16,
**characterized in that**
the passage (11, 23) provided for receiving an implement (19) has a stiffened portion which in particular extends along a peripheral section extending at the outer side of a curvature with a curvature of the disposable sheath and/or along a longitudinal section of the disposable sheath; and/or
**in that** one or two additional working passages are provided; and/or in that at least one fixing device (37) is provided by which the position of the implement (19) is temporarily fixable relative to the disposable sheath, with the fixing device preferably having a fixing screw (37).

18. A disposable sheath in accordance with any one of the preceding claims,
**characterized in that**
the proximal end of the flushing passage (13) has a connection device (59, 63) for connection to a pump or to a flushing container, with the connection device in particular having a flexible hose, a connection flange (59) and/or a thread (63).

19. A disposable sheath in accordance with any one of the preceding claims,
**characterized in that**
the flushing passage has at least one closure device (39, 41, 51) for a temporary closure of the flushing passage or has at least one reception device for receiving a reusable closure device (43), with a separation device, in particular a flexible membrane (49), preferably being provided for separating the reusable closure device (43) from the flushing passage (13).

20. A disposable sheath in accordance with any one of the preceding claims,
**characterized in that**
at least one suction passage (15) is provided for sucking off liquid or gas at the distal end of the disposable sheath,
with the proximal end of the flushing passage (15) preferably having a connection device (59, 63) for connection to a pump, with the connection device in particular having a flexible hose, a connection flange (59) and/or a thread (63).

21. A disposable sheath in accordance with claim 20,
**characterized in that**
the suction passage (15) has at least one closure device (39, 41, 51) for the temporary closure of the suction passage or has at least one reception device for receiving a reusable closure device (43), with a separation device, in particular a flexible membrane (49), preferably being provided for separating the reusable closure device (43) from the suction passage (15); and/or
**in that** the distal opening of the flushing passage (13) and the distal opening of the suction passage (15) are arranged opposite one another with respect to the distal opening of the endoscope passage (11,21).

22. A disposable sheath in accordance with any one of the preceding claims,
**characterized in that**
the disposable sheath has a substantially circular or oval external cross-section or an external cross-section corresponding to the cross-section of the human nasal passage; and/or
**in that** the disposable sheath is formed from a flexible material, in particular from an elastic material, preferably from a plastic; and/or in that the disposable sheath is configured as elastic with respect to its total periphery or with respect to a peripheral section (89, 91), and indeed along a longitudinal section or along the total length of the disposable sheath.

23. A disposable sheath in accordance with any one of the preceding claims,
**characterized in that**
the endoscope passage (21), the working passage (22) and/or the endoscope/working passage (11) is/are laterally open along one or more longitudinal sections of the disposable sheath; and/or
**in that** the disposable sheath is formed from a transparent material; and/or
**in that** at least the peripheral region of the disposable sheath, preferably the total disposable sheath, is formed in one piece; and/or
**in that** the passages (11, 13, 15, 21, 23) contained in the disposable sheath open in an aligned manner towards one another at the distal end of the disposable sheath; and/or
**in that** the passages (11, 13, 15, 21, 23) extend substantially in parallel with one another within the disposable sheath.

24. A disposable sheath in accordance with any one of the preceding claims,
**characterized in that**
a widened closure portion (65) is provided at the proximal end of the disposable sheath for preventing a complete penetration of the disposable sheath into the body of a patient; and/or
**in that** the disposable sheath has a length between 50 and 130 cm; and/or
the endoscope (17) is a compact endoscope without its own working passage and in particular without its own flushing or suction passage; and/or
**in that** the endoscope (17) has at least one light/image transmission passage (85, 87); and/or
**in that** the endoscope (17) has at least one Bowden cable.

## Revendications

1. Gaine à jeter pour un usage unique, comprenant au moins un canal pour endoscope (11, 21) destiné à recevoir un endoscope (17), dans laquelle il est prévu au moins un canal de rinçage (13, 27) séparé du canal pour endoscope (11, 21) pour la transmission d'un liquide ou d'un gaz vers l'extrémité distale de la gaine à jeter,
**caractérisée en ce que**
il est prévu au moins un moyen de fixation grâce auquel la position de l'extrémité distale de l'endoscope peut être fixée par rapport à la gaine à jeter, ledit moyen de fixation comprenant une collerette de rétrécissement à l'extrémité distale du canal pour endoscope, qui assure une coopération de force avec l'endoscope (17) et ainsi une friction augmentée, de sorte qu'un déplacement de l'endoscope prisonnier dans la collerette de rétrécissement est rendu difficile, et
**en ce qu'**il est prévu à l'extrémité distale de la gaine à jeter et dans la région d'une ouverture de sortie du canal de rinçage (13, 27) un dispositif de déflexion (33) pour défléchir un liquide ou un gaz de rinçage transmis via le canal de rinçage (13, 27).

2. Gaine à jeter selon la revendication 1,
**caractérisée en ce que** le canal pour endoscope est réalisé sous forme de canal pour endoscope/travail destiné à recevoir additionnellement un appareil de travail (19), dans laquelle le canal pour endoscope/travail (11) présente une section intérieure essentiellement ovale et/ou au moins une et de préférence deux barrettes de guidage opposées (25, 31) pour guider l'endoscope (17) et l'appareil de travail (19).

3. Gaine à jeter selon la revendication 1,
**caractérisée en ce que**
le canal pour endoscope (21) est réalisé pour recevoir uniquement l'endoscope (17), et **en ce qu'**il est prévu au moins un canal de travail (23), séparé du canal pour endoscope (21), pour la réception d'un appareil de travail (19),
dans laquelle le canal pour endoscope (21) et/ou le canal de travail (23) présentent en particulier chacun une section intérieure essentiellement circulaire.

4. Gaine à jeter selon l'une des revendications précédentes, **caractérisée en ce que** la gaine à jeter comprend au moins un moyen de couplage extérieur (67, 93, 99, 101, 103, 105, 107) pour le couplage d'un appareil de travail (19) contre le côté extérieur de la gaine à jeter.

5. Gaine à jeter selon la revendication 4,
**caractérisée en ce que** le moyen de couplage extérieur comprend un moyen de liaison à encliquetage (67, 101, 102, 105), un moyen de couplage magnétique (99, 107), un moyen de couplage agrippant, un moyen de liaison par collage et/ou un moyen de liaison à languette-et-rainure (93),
dans laquelle le moyen de liaison à encliquetage comprend de préférence au moins une goulotte d'encliquetage élastique (67, 101, 103) ou une pince à encliquetage élastique (105) pour enserrer l'appareil de travail (19), et/ou
dans laquelle de préférence le moyen de couplage magnétique (99, 107)
- comprend un aimant permanent,
- s'étend en direction longitudinale de la gaine à jeter en continu ou uniquement le long d'un ou de plusieurs tronçons longitudinaux,
- est prévu à une extrémité distale de la gaine à jeter,
- est réparti sous forme de poudre à l'intérieur de la gaine à jeter, et/ou
- est réalisé sous forme d'unité réutilisable interchangeable.

6. Gaine à jeter selon l'une des revendications 4 et 5,
**caractérisée en ce que**
le moyen de couplage extérieur (67, 93, 99, 101, 103, 105, 107) est prévu uniquement le long d'un ou de plusieurs tronçons périphériques de la gaine à jeter, et/ou
**en ce que** l'appareil de travail (19) comprend au moins un moyen de couplage antagoniste (95, 111) qui coopère avec le moyen de couplage extérieur (67, 93, 99, 101, 103, 105, 107), et/ou
**en ce que** l'appareil de travail (19) est déplaçable par rapport au moyen de couplage extérieur (67, 93, 99, 101, 103, 105, 107) en direction longitudinale de la gaine à jeter.

7. Gaine à jeter selon l'une des revendications 4 à 6,
**caractérisée en ce qu'**il est prévu un moyen de fixation grâce auquel la position de l'appareil de travail (19) peut être fixée par rapport à la direction longitudinale de la gaine à jeter,
de préférence ledit moyen de fixation
- est prévu à l'extrémité distale de la gaine à jeter,
- comprend un aimant permanent (99), un électroaimant ou un système à accrochage à l'extrémité distale de la gaine à jeter,
- comprend un aimant permanent (99) au niveau d'un tronçon longitudinal de la gaine à jeter, et/ou
- comprend une pince de fixation susceptible d'être bloquée à l'extrémité proximale de la gaine à jeter.

8. Gaine à jeter selon l'une des revendications 4 à 7,
**caractérisée en ce que**
la gaine à jeter comporte deux surélévations périphériques (81) qui entourent latéralement le moyen de couplage extérieur et qui incluent un tronçon périphérique (83) à section concave pour la réception de l'appareil de travail (19), et/ou
**en ce que** la gaine à jeter comprend plusieurs, en particulier deux ou trois moyens de couplage extérieur (67, 93, 99, 101, 103, 105, 107) pour un couplage d'un nombre correspondant d'appareils de travail (19), et/ou
**en ce qu'**au moins un moyen de couplage extérieur (67, 99) est réalisé pour un couplage d'un appareil de travail (19) tel que l'extrémité distale de l'appareil de travail est agencée et en particulier fixée au niveau d'un tronçon longitudinal entre l'extrémité distale et l'extrémité proximale de la gaine à jeter.

9. Gaine à jeter selon l'une des revendications 4 à 8,
**caractérisée en ce que**
il est en outre prévu un tuyau de couplage (97) qui, pour le couplage sur la gaine à jeter, comprend au moins un moyen de couplage antagoniste (95, 111) coopérant avec le moyen de couplage extérieur (67, 93, 107),
dans laquelle le tuyau de couplage (97) est en particulier prévu sous forme de pièce à jeter et/ou est formé pour la réception de l'appareil de travail (19).

10. Gaine à jeter selon l'une des revendications 4 à 9,
**caractérisée en ce que**
il est prévu à titre d'appareil de travail un tuyau d'aspiration/rinçage (19) avec au moins un canal d'aspiration/rinçage (13, 15),
dans laquelle de préférence le tuyau d'aspiration/rinçage
- est susceptible d'être fixé au moyen d'un dispositif de fixation distal (103), par rapport à la direction longitudinale de la gaine à jeter, de telle façon que l'extrémité distale du canal d'aspiration/rinçage est à une distance prédéterminée de l'extrémité distale de la gaine à jeter ou de l'endoscope (17) reçu dans celle-ci, et/ou
- est susceptible d'être fixé au moyen d'un dispositif extérieur de couplage proximal (101) et d'un dispositif de couplage distal (103), par rapport à la direction longitudinale de la gaine à jeter de telle façon que lors d'une courbure de la gaine à jeter le tuyau d'aspiration/rinçage est tendu ou allongé, et/ou
- est susceptible d'être allongé élastiquement direction longitudinale, et/ou
- comprend à l'extrémité distale un moyen de déflexion pour défléchir un liquide de rinçage ou un gaz de rinçage.

11. Gaine à jeter selon l'une des revendications 4 à 10,
**caractérisée en ce que**
la gaine à jeter comprend au moins un moyen de couplage magnétique (107) pour un couplage d'un appareil de travail (19) contre le côté extérieur de la gaine à jeter ainsi que deux moyens de couplage extérieur (67) qui entourent le moyen de couplage magnétique, pour un couplage d'un tuyau d'aspiration/rinçage (19) respectif, et/ou
**en ce que** le canal pour endoscope (21) est réalisé pour recevoir uniquement l'endoscope (17).

12. Gaine à jeter selon l'une des revendications précédentes, **caractérisée en ce qu'**au moins un tronçon périphérique, limitant le canal pour endoscope (21), de la gaine à jeter est réalisé sous forme d'un tronçon de transmission de couplage (83, 113) qui permet, pour un couplage extérieur d'un appareil de travail (19) sur l'endoscope (17) qui se trouve dans la gaine à jeter, une coopération d'un moyen de maintien magnétique (109) de l'endoscope avec un moyen antagoniste magnétique (111) de l'appareil de travail.

13. Gaine à jeter selon la revendication 12,
**caractérisée en ce que** le tronçon de transmission de couplage de la gaine à jeter présente un amincissement (83) dans la gaine ou une ouverture périphérique (113), grâce auquel/à laquelle le moyen de maintien magnétique (109) et le moyen antagoniste magnétique (111) coopèrent, et/ou
**en ce qu'**il est prévu le long de la direction longitudinale de la gaine à jeter un tronçon de transmission de couplage continu ou plusieurs tronçons de transmission de couplage séparés (83, 113), et/ou en ce qu'il est prévu un tronçon de transmission de couplage (83, 113) à une extrémité distale de la gaine à jeter.

14. Gaine à jeter selon l'une des revendications 12 et 13,
**caractérisée en ce que**
il est prévu à titre d'appareil de travail un tuyau d'aspiration/rinçage (19) avec au moins un canal d'aspiration/rinçage (13, 15), et/ou en ce que la gaine à jeter comporte deux surélévations périphériques (81), qui entourent latéralement le tronçon de transmission de couplage (83, 113) et qui incluent un tronçon périphérique à section concave pour la réception de l'appareil de travail, et/ou
**en ce qu'**il est en outre prévu un tuyau de couplage (97), qui est réalisé pour recevoir l'appareil de travail (19) et le moyen antagoniste magnétique (111), ledit tuyau de couplage (97) étant en particulier prévu sous forme de pièce à jeter.

15. Gaine à jeter selon l'une des revendications 4 à 14,
**caractérisée en ce que** la gaine à jeter comprend deux canaux d'aspiration/rinçage (13, 15), qui sont agencés par rapport au canal pour endoscope (21) de telle façon que la gaine à jeter comporte, sur son côté extérieur entre les canaux d'aspiration/rinçage, un tronçon périphérique à section concave (83) pour la réception de l'appareil de travail (19).

16. Gaine à jeter selon la revendication 15,
**caractérisée en ce que**
il est prévu un moyen de couplage extérieur (67, 93, 99, 101, 103, 105, 107) ou un tronçon de transmission de couplage (83, 113) au niveau du tronçon périphérique concave, et/ou
la gaine à jeter est réalisée de manière élastique au moins le long du tronçon périphérique concave (83), et/ou
les axes longitudinaux (71, 73) des canaux d'aspiration/rinçage (13, 15) occupent, par rapport à l'axe longitudinal (75) du canal pour endoscope (21), en section transversale un angle entre 30° et 120°, en particulier un angle d'environ 45°, 60° ou 90°, l'un par rapport à l'autre.

17. Gaine à jeter selon l'une des revendications 2 à 16,
**caractérisée en ce que**
le canal (11, 23) prévu pour la réception d'un appareil de travail (19) comporte une rigidification, qui s'étend en particulier le long d'un tronçon périphérique de la gaine à jeter qui, lors d'une courbure de la gaine à jeter, se trouve du côté extérieur de la courbure, et/ou le long d'un tronçon longitudinal de la gaine à jeter, et/ou
il est prévu un ou deux canaux de travail additionnel(s), et/ou il est prévu au moins un moyen de fixation (37) grâce auquel la position de l'appareil de travail (19) par rapport à la gaine à jeter est susceptible d'être fixée temporairement, et le moyen de fixation comprend au de préférence une vis de fixation (37).

18. Gaine à jeter selon l'une des revendications précédentes, **caractérisée en ce que** l'extrémité proximale du canal de rinçage (13) comprend un moyen de liaison (59, 63) pour le raccordement à une pompe ou à un récipient le rinçage, dans laquelle le moyen de liaison comprend en particulier un tuyau flexible, une bride de raccordement (59) et/ou un pas de vis (63).

19. Gaine à jeter selon l'une des revendications précédentes, **caractérisée en ce que** le canal de rinçage comprend au moins un moyen d'obturation (39, 41, 51) pour l'obturation temporaire du canal de rinçage, ou au moins un moyen de réception pour la réception d'un moyen d'obturation réutilisable (43), dans laquelle il est prévu au moins un dispositif de séparation, en particulier une membrane flexible (49) pour la séparation du moyen d'obturation réutilisable (43) vis-à-vis du canal de rinçage (13).

20. Gaine à jeter selon l'une des revendications précédentes, **caractérisée en ce qu'**il est prévu au moins un canal d'aspiration (15) pour aspirer un liquide ou un gaz à l'extrémité distale de la gaine à jeter, dans laquelle l'extrémité proximale du canal d'aspiration (15) comprend au moins un moyen de liaison (59, 63) pour le raccordement à une pompe, dans laquelle le moyen de liaison comprend en particulier un tuyau flexible, une bride de raccordement (59) et/ou un pas de vis (63).

21. Gaine à jeter selon la revendication 20,
**caractérisée en ce que**
le canal d'aspiration (15) comprend au moins un moyen d'obturation (39, 41, 51) pour l'obturation temporaire du canal d'aspiration, ou au moins un moyen de réception pour la réception d'un moyen d'obturation réutilisable (43), et il est de préférence prévue un moyen de séparation, en particulier une membrane flexible (49) pour la séparation du moyen d'obturation réutilisable (43) vis-à-vis du canal d'aspiration (15), et/ou
**en ce que** l'ouverture distale du canal de rinçage (13) et l'ouverture distale du canal d'aspiration (15) sont agencées à l'opposé l'une de l'autre par rapport à l'ouverture distale du canal pour endoscope (11,21).

22. Gaine à jeter selon l'une des revendications précédentes, **caractérisée en ce que**
la gaine à jeter présente une section extérieure sensiblement circulaire ou ovale, ou une section extérieure correspondre à la section du canal nasal humain, et/ou
la gaine à jeter est formée en un matériau flexible, en particulier en un matériau élastique et de préférence en matière plastique, et/ou
la gaine à jeter est réalisée de manière élastique pour ce qui concerne la totalité de sa périphérie ou pour ce qui concerne un tronçon périphérique (89, 91), et cela le long d'un tronçon longitudinal ou le long de la totalité de la longueur de la gaine à jeter.

23. Gaine à jeter selon l'une des revendications précédentes, **caractérisée en ce que**
le canal pour endoscope (21), le canal de travail (22) et/ou le canal pour endoscope/travail (11) est ouvert latéralement le long d'un ou plusieurs tronçons longitudinaux de la gaine à jeter, et/ou
la gaine à jeter est formée en un matériau transparent, et/ou
au moins la région périphérique de la gaine à jeter et de préférence la totalité de la gaine à jeter est réalisée d'un seul tenant, et/ou les canaux (11, 13, 15, 21, 23) contenus dans la gaine à jeter débouchent sensiblement en affleurement les uns par rapport aux autres à l'extrémité distale de la gaine à jeter, et/ou
les canaux (11, 13, 15, 21, 23) s'étendent sensiblement parallèlement les uns aux autres à l'intérieur de la gaine à jeter.

24. Gaine à jeter selon l'une des revendications précédentes, **caractérisée en ce que**
pour empêcher une pénétration totale de la gaine à jeter dans le corps d'un patient, il est prévu un élargissement terminal (65) à l'extrémité proximale de la gaine à jeter, et/ou
la gaine à jeter a une longueur entre 50 et 130 centimètres, et/ou l'endoscope (17) est un endoscope compact dépourvu de propre canal de travail, et en particulier dépourvu de propre canal de rinçage ou d'aspiration, et/ou
l'endoscope (17) comprend au moins un canal de transmission de lumière/image (85, 87), et/ou
l'endoscope (17) comprend au moins un câble Bowden.
